# EUROPEAN PATENT APPLICATION

(11) **EP 1 902 663 A1**
(43) Date of publication of application: **26.03.2008**
(21) Application number: 06780838.6
(22) Date of filing: 06.07.2006
(51) Int. Cl.: A61B 1/00, A61B 5/07

(54) **APPARATUS FOR PLACING CAPSULE TYPE MEDICAL DEVICE, APPARATUS FOR PLACING CAPSULE ENDOSCOPE IN THE BODY AND CAPSULE TYPE MEDICAL DEVICE FOR PLACEMENT**

(30) Priority: 08.07.2005 JP 2005200886; 19.07.2005 JP 2005209089
(71) Applicant: OLYMPUS MEDICAL SYSTEMS CORP., Shibuya-ku Tokyo 151-0072 (JP); Olympus Corporation, Tokyo 151-0072 (JP)
(72) Inventor: TAKIZAWA, Hironobu, c/o OLYMPUS MED. SYSTEMS CORP., Tokyo 151-0072 (JP); TANAKA, Shinsuke c/o OLYMPUS MED. SYSTEMS CORP., Tokyo 151-0072 (JP); HIRAKAWA, Katsumi c/o OLYMPUS MED. SYSTEMS CORP., Tokyo 151-0072 (JP); UCHIYAMA, Akio c/o OLYMPUS MED. SYSTEMS CORP., Tokyo 151-0072 (JP); YOKOI, Takeshi c/o OLYMPUS MED. SYSTEMS CORP., Tokyo 151-0072 (JP)
(74) Representative: von Hellfeld, Axel
(86) International application number: PCT/JP2006/313512
(87) International publication number: WO 2007/007648

(57) **Abstract**

An already-existing and general-purpose capsule endoscope for alimentary canal is enabled to be used to monitor a body cavity by directly diverting the already-existing and general-purpose capsule endoscope to a body-cavity-indwelling capsule endoscope in a simple and secure manner. An indwelling device for an encapsulated medical device of the invention includes a latching portion 203 which fixes a capsule endoscope 300 and a retaining unit 202 which mounts and retains a capsule endoscope 300. Therefore, the capsule endoscope 300 and the latching portion 203 are integral with each other only by mounting and retaining the capsule endoscope 300 to and in the retaining unit 202, and then the capsule endoscope 300 can be placed along with the latching portion 203 by setting the latching portion 203 to a desired region in the body cavity to fix the latching portion 203 to a body cavity tissue by endoscopic treatment. Accordingly, the already-existing and general-purpose capsule endoscope 300 for alimentary canal can be used to monitor a body cavity by directly diverting the already-existing and general-purpose capsule endoscope to the body-cavity-indwelling capsule endoscope in the simple and secure manner.

## Description

### TECHNICAL FIELD

The present invention relates to an indwelling device for an encapsulated medical device and an in vivo indwelling device for a capsule endoscope which utilize a capsule endoscope for alimentary canal such as a capsule endoscope for small intestine as a body cavity indwelling capsule, and a capsule-indwelling-type medical device in which the general purpose capsule endoscope or a body-cavity-indwelling capsule endoscope for indwelling purpose only is used.

### BACKGROUND ART

Recently, a swallowable capsule endoscope has been developed in the field of the endoscope. The capsule endoscope has both an imaging function and a wireless communication function. After the capsule endoscope is swallowed from a mouth of a patient to observe a body cavity, the capsule endoscope moves through the inside of an organ such as the esophagus, the stomach, and the small intestine according to peristaltic movement of the organ, and the capsule endoscope sequentially takes an image of the organ until naturally discharged from a human body (for example see Patent Document 1).

Data of the image which is taken in the body by the capsule endoscope while the capsule endoscope moves in the body cavity is sequentially transmitted to the outside of the body through the wireless communication and stored in a memory provided in a receiver outside the body. A doctor or a nurse can makes a diagnosis based on the image displayed on the screen from the image data stored in the memory.

On the other hand, the development of the endoscope technology enables an endoscopic operation such as endoscopic mucosa resection (EMR) and endoscopic submucosa detachment (ESD). Although bleeding is stopped in an operated region after the endoscopic operation, it is necessary to monitor the presence or absence of the bleeding because there is a risk of bleeding during nighttime hours. Therefore, there is proposed a method of monitoring the inside of the body cavity with the capsule endoscope which is configured to be able to be placed at a desired position in the body cavity (for example see Patent Document 2).

Furthermore, for example, Patent Document 3 discloses a technique of fixing a medical capsule for detecting pH in the body cavity to a target region in the body cavity.

Patent Document 1: Japanese Patent Application Laid-Open No. 2003-19111
Patent Document 2: US Patent No.2002/0042562
Patent Document 3: Japanese Patent Application Laid-Open No. S58-19232

### DISCLOSURE OF THE INVENTION

### PROBLEM TO BE SOLVED BY THE INVENTION

In the conventional technique disclosed in Patent Document 2, the capsule endoscope includes an attachment which can be detachably attached to a living tissue, and thereby the capsule endoscope can be placed in the body cavity. However, the capsule endoscope is originally configured to obtain information in the body cavity while passing through an alimentary canal, so that it is difficult that the attachment is securely attached to the capsule endoscope without having an influence on the capsule endoscope. Therefore, it is necessary to originally produce a capsule endoscope for indwelling use only.

Furthermore, in the conventional technique disclosed in Patent Document 2, the capsule endoscope includes plural fasteners, and the capsule endoscope is latched to a body cavity tissue at plural points. However, the capsule endoscope is simply hung and latched to the body cavity tissue, so that moment acts on a front edge of the capsule endoscope to easily swing the capsule endoscope in the latched and placed state. Therefore, an observation visual field or an orientation of an observation axis is shifted in the capsule endoscope having an observation optical system, which loses the continuous and stable monitoring function to the region to be monitored.

In the conventional technique disclosed in Patent Document 3, the medical capsule includes plural needle-shape members having a keen portion, and the needle-shape members are inserted into body cavity tissues so as to intersect each other, which allows the medical capsule to be latched at plural points. However, it is necessary to modify the inner structure of the medical capsule in a complicated manner, so that the medical capsule disclosed in Patent Document 3 is hardly applied to the capsule endoscope having the observation optical system.

In view of the foregoing, it is an object of the invention to provide an indwelling device for an encapsulated medical device, an in vivo indwelling device for a capsule endoscope, and a capsule-indwelling-type medical device, in which the already-existing and general-purpose capsule endoscope such as the alimentary canal capsule endoscope can be used to monitor the body cavity by directly diverting the already-existing and general-purpose capsule endoscope to a body-cavity-indwelling capsule endoscope in a simple and secure manner.

It is another object of the invention to provide an indwelling device for an encapsulated medical device and a capsule-indwelling-type medical device, which can place the encapsulated medical device such that an orientation of the observation visual field is not shifted with simple structure, and can be used to monitor the body cavity while continuously kept in the stable state.

### MEANS FOR SOLVING PROBLEM

In order to solve the above problems to achieve the objects, an indwelling device for an encapsulated medical device according to the present invention includes a latching portion for fixing an encapsulated medical device to a body cavity tissue, the encapsulated medical device being introduced into a body cavity of a subject to obtain information of the body cavity, and to wirelessly transmit and output the information of the body cavity to an outside of the subject; and a retaining unit that is provided with the latching portion to mount and retain the encapsulated medical device.

In the indwelling device for an encapsulated medical device according to the present invention, the retaining unit has a structure which integrally retains a capsule endoscope as the encapsulated medical device including an observation optical system in a state of being in a surface contact with a peripheral surface of the capsule endoscope.

In the indwelling device for an encapsulated medical device according to the present invention, the retaining unit includes a substantially cylindrical portion having a radius which is substantially the same as the capsule endoscope.

In the indwelling device for an encapsulated medical device according to the present invention, the retaining unit is formed in a cap shape.

In the indwelling device for an encapsulated medical device according to the present invention, the retaining unit includes a hood portion for the observation optical system.

In the indwelling device for an encapsulated medical device according to the present invention, the hood portion is located outside an observation visual field of the observation optical system.

In the indwelling device for an encapsulated medical device according to the present invention, the hood portion has an abutting portion where the capsule endoscope abuts when mounted.

In the indwelling device for an encapsulated medical device according to the present invention, the hood portion is formed by a transparent member.

In the indwelling device for an encapsulated medical device according to the present invention, the hood portion is located within the observation visual field of the observation optical system.

In the indwelling device for an encapsulated medical device according to the present invention, the hood portion has a dome shape which covers a whole of the observation visual field of the observation optical system.

In the indwelling device for an encapsulated medical device according to the present invention, a dirt protective coating is applied to an outer surface of the hood portion.

In the indwelling device for an encapsulated medical device according to the present invention, the dirt protective coating is a water-repellent coating.

In the indwelling device for an encapsulated medical device according to the present invention, the dirt protective coating is a hydrophilic coating.

In the indwelling device for an encapsulated medical device according to the present invention, the dome shape portion of the hood portion has optical characteristics which functions with respect to the observation optical system.

In the indwelling device for an encapsulated medical device according to the present invention, the retaining unit is integral with the latching portion which is formed at a position within the observation visual field of the observation optical system.

In the indwelling device for an encapsulated medical device according to the present invention, the latching portion is a clip which is provided so as to be coupled to the retaining unit by a string member, the clip being fixed to a body cavity tissue by an endoscopic treatment tool.

In the indwelling device for an encapsulated medical device according to the present invention, the latching portion is a hole portion which is formed so as to be integral with the retaining unit, the hole portion being fixed to the body cavity tissue by an endoscopic fixture.

In the indwelling device for an encapsulated medical device according to the present invention, the latching portion is a suction portion which is formed so as to be integral with the retaining unit, the suction portion sucking a part of the body cavity tissue to be fixed by the endoscopic fixture.

In the indwelling device for an encapsulated medical device according to the present invention, the latching portion is an annular string member which is provided so as to be coupled to the retaining unit, the annular string member being fixed to the body cavity tissue by the endoscopic fixture.

In the indwelling device for an encapsulated medical device according to the present invention, the latching portion is a mesh member which is provided so as to be coupled to the retaining unit, an arbitrary point of the mesh member being fixed to the body cavity tissue by the endoscopic fixture.

In the indwelling device for an encapsulated medical device according to the present invention, a plurality of latching portions are provided so as to be located at different points of the retaining member.

In the indwelling device for an encapsulated medical device according to the present invention, the plurality of latching portions have differences in one of size and shape.

A capsule-indwelling-type medical device according to the present invention includes the indwelling device for an encapsulated medical device according to any one of claims 1 to 22; and an encapsulated medical device that is mounted and retained in a retaining unit of the indwelling device for an encapsulated medical device, introduced into a body cavity, fixed to a body cavity tissue by a latching portion, and obtains information of the body cavity of a subject to wirelessly transmit and output the information of the body cavity to an outside of the subject.

An in vivo indwelling device for a capsule endoscope according to the present invention includes a retainer that has an engaging and retaining unit capable of engaging with an exterior of a capsule endoscope to retain the capsule endoscope; an opening portion that is formed in the retainer so as to be capable of securing an observation visual field of the capsule endoscope retained by the engaging and retaining unit; and an attachment unit that is provided in an outer surface of the retainer, and can be attached to an organism wall.

In the in vivo indwelling device for a capsule endoscope according to the present invention, the opening portion is formed at one end portion of the retainer, and the attachment unit is provided at the other end portion of the retainer.

An indwelling device for an encapsulated medical device according to the present invention includes a retaining member that mounts and retains an encapsulated medical device, the encapsulated medical device being introduced into a body cavity to obtain information of a body cavity; and a latching member that is provided so as to be coupled to the retaining member, and has a plurality of latching portions which are latched to a body cavity tissue with tension at positions located outside a projection plane of the encapsulated medical device with respect to the body cavity tissue, the latching portions holding the encapsulated medical device at the positions.

In the indwelling device for an encapsulated medical device according to the present invention, the latching member has the plurality of latching portions which are latched to the body cavity tissue so that a generatrix of a capsule endoscope as the encapsulated medical device including an observation optical system comes into line contact with the body cavity tissue in a desired direction.

In the indwelling device for an encapsulated medical device according to the present invention, the latching member has the plurality of latching portions whose latching positions are set so as to prevent an orientation of an observation visual field of the observation optical system from deviating.

In the indwelling device for an encapsulated medical device according to the present invention, the latching member has the plurality of latching portions whose latching positions are set so as to put an observation axis of the observation optical system therebetween.

In the indwelling device for an encapsulated medical device according to the present invention, the latching member has the plurality of latching portions whose latching positions are set so as to offset moments acting on the encapsulated medical device from the respective latching positions.

In the indwelling device for an encapsulated medical device according to the present invention, the latching member is formed by an elastic material which allows a flexible folding of the latching member around the retaining member in introducing into the body cavity, and spreading and returning thereof to an original static shape in releasing into the body cavity.

In the indwelling device for an encapsulated medical device according to the present invention, the latching member is formed in a grip shape.

In the indwelling device for an encapsulated medical device according to the present invention, the latching member is formed in a rod shape.

In the indwelling device for an encapsulated medical device according to the present invention, the latching member is coupled so as to be biased to one side face of the retaining member in a tangential manner.

In the indwelling device for an encapsulated medical device according to the present invention, the latching member is rotatably coupled to the retaining member.

In the indwelling device for an encapsulated medical device according to the present invention, the latching member includes a banding member having a non-return structure.

In the indwelling device for an encapsulated medical device according to the present invention, the latching member is formed by a guide member which, having a non-return structure, is arranged in an observation axis of the observation optical system, and to which the retaining member is movably coupled.

In the indwelling device for an encapsulated medical device according to the present invention, the latching portion is a portion which is latched to the body cavity tissue by an endoscopic fixture.

In the indwelling device for an encapsulated medical device according to the present invention, the latching portion is formed in a multistage so that a latching performed by the endoscopic fixture can be freely selected in the latching member.

In the indwelling device for an encapsulated medical device according to the present invention, the latching portion includes a latching portion which has a self-latching function with respect to the body cavity tissue, and which is directly latched to the body cavity tissue by an endoscopic treatment.

A capsule-indwelling-type medical device according to the present invention includes the indwelling device for an encapsulated medical device according to any one of claims 26 to 40; and an encapsulated medical device that is mounted to and retained in a retaining member of the indwelling device for an encapsulated medical device, introduced into a body cavity from an oral cavity, fixed to a body cavity tissue by a latching portion of a latching member, and obtains information of the body cavity of a subject to wirelessly transmit and output the information of the body cavity to an outside of the subject.

The capsule-indwelling-type medical device according to the present invention includes an encapsulated medical device that has an observation optical system; and a latching member that has a plurality of latching portions provided outside a visual field of the observation optical system in the encapsulated medical device, the latching portions attaching the encapsulated medical device to a body cavity tissue.

In the capsule-indwelling-type medical device according to the present invention, the encapsulated medical device includes a setting portion in a lowermost portion thereof, the setting portion placing the encapsulated medical device to the body cavity tissue, and the latching member includes a first latching member having a first latching portion which is provided at a position upwardly away from the setting portion in the encapsulated medical device and attached to the body cavity tissue by an attachment member; and a second latching member having a second latching portion which is provided at a position upwardly away from the setting portion and displaced from the first latching portion, the second latching portion being attached to the body cavity tissue by the attachment member.

A capsule-indwelling-type medical device according to the present invention includes an encapsulated medical device that is introduced into a body cavity to obtain information of the body cavity of a subject; and a latching member that is provided so as to be coupled to the encapsulated medical device, and has a plurality of latching portions which are latched to a body cavity tissue with tension at positions located outside a projection plane of the encapsulated medical device with respect to the body cavity tissue, the latching portions holding the encapsulated medical device at the positions.

In the capsule-indwelling-type medical device according to the present invention, the encapsulated medical device is a capsule endoscope including an observation optical system.

In the capsule-indwelling-type medical device according to the present invention, the latching member has the plurality of latching portions which are latched to the body cavity tissue so that a generatrix of the encapsulated medical device comes into line contact with the body cavity tissue in a desired direction.

In the capsule-indwelling-type medical device according to the present invention, the latching member has the plurality of latching portions whose latching positions are set so as to prevent an orientation of an observation visual field of the observation optical system from deviating.

In the capsule-indwelling-type medical device according to the present invention, the latching member has the plurality of latching portions whose latching positions are set so as to put an observation axis of the observation optical system therebetween.

In the capsule-indwelling-type medical device according to the present invention, the latching member has the plurality of latching portions whose latching portions are set so as to offset moments acting on the encapsulated medical device from the respective latching positions.

In the capsule-indwelling-type medical device according to the present invention, the latching member is formed by an elastic material which allows a flexible folding of the latching member around the encapsulated medical device in introducing into the body cavity, and spreading and returning thereof to an original static shape in releasing into the body cavity.

In the capsule-indwelling-type medical device according to the present invention, the latching member is formed in a grip shape.

In the capsule-indwelling-type medical device according to the present invention, the latching member is formed in a rod shape.

In the capsule-indwelling-type medical device according to the present invention, the latching member is formed by a mesh member.

In the capsule-indwelling-type medical device according to the present invention, the latching member is formed by a sheet-shape member.

In the capsule-indwelling-type medical device according to the present invention, the latching member is coupled so as to be biased to one side face of the encapsulated medical device in a tangential manner.

In the capsule-indwelling-type medical device according to the present invention, the latching member is arranged so that a joint portion is located on a body cavity tissue side.

In the capsule-indwelling-type medical device according to the present invention, the latching member is arranged so that a joint portion is located on a side opposite to the body cavity tissue.

In the capsule-indwelling-type medical device according to the present invention, the latching member includes a banding member having a non-return structure.

In the capsule-indwelling-type medical device according to the present invention, the latching member is formed by a guide member which, having a non-return structure, is arranged in an observation axis of the observation optical system, and to which the encapsulated medical device is movably coupled.

In the capsule-indwelling-type medical device according to the present invention, the latching portion is a portion which is latched to the body cavity tissue by an endoscopic fixture.

In the capsule-indwelling-type medical device according to the present invention, the latching portion is formed in a multistage so that a latching performed by the endoscopic fixture can be freely selected in the latching member.

In the capsule-indwelling-type medical device according to the present invention, the latching portion includes a latching portion which has a self-latching function with respect to the body cavity tissue, and is directly latched to the body cavity tissue by an endoscopic treatment.

### EFFECT OF THE INVENTION

According to the invention, the indwelling device for an encapsulated medical device, the in vivo indwelling device for a capsule endoscope, and the capsule-indwelling-type medical device include the latching portion for fixing the encapsulated medical device such as the capsule endoscope to the body cavity tissue; and the retaining unit that mounts and retains the encapsulated medical device. Therefore, the encapsulated medical device can be integral with the latching portion only by mounting and retaining the encapsulated medical device to and in the retaining unit, and then the encapsulated medical device can be placed along with the retaining unit by fixing the latching portion to the body cavity tissue by the endoscopic treatment in the desired region in the body cavity. Consequently, the invention has an effect of being able to use the already-existing and general-purpose capsule endoscope such as the alimentary canal capsule endoscope as the body-cavity-indwelling capsule endoscope to monitor the body cavity by directly diverting the already-existing and general-purpose capsule endoscope to the body-cavity-indwelling capsule endoscope in a simple and secure manner.

According to the present invention, the indwelling device for an encapsulated medical device and the capsule-indwelling-type medical device include the latching member having the plural latching portions which are provided so as to be coupled to the retaining member for mounting and retaining the encapsulated medical device or the encapsulated medical device, the latching portions being latched to the body cavity tissue with tension at positions located outside the projection plane of the encapsulated medical device with respect to the body cavity tissue, the latching portions holding the encapsulated medical device at the positions. Therefore, the encapsulated medical device comes into line contact with the body cavity tissue so that the generatrix of the encapsulated medical device is arranged in the desired direction, and the encapsulated medical device can be latched so as to prevent the orientation of the observation visual field or the observation axis from deviating. Consequently, the invention has an effect of being able to place the encapsulated medical device so that the orientation of the observation visual field is not deviated with a simple structure, and of being able to be used to monitor the body cavity while continuously kept in the stable state.

### BRIEF DESCRIPTION OF THE DRAWINGS

FIG. 1 is a sectional construction drawing showing a configuration example of a capsule-indwelling-type medical device according to a first embodiment of the invention;
FIG. 2 is a schematic drawing showing a configuration example of a wireless capsule-indwelling-type medical system;
FIG. 3 is a schematic front view showing a state in which a capsule-indwelling-type medical device is attached to an endoscope;
FIG. 4A is a schematic drawing showing a state in which the endoscope is introduced into a body cavity;
FIG. 4B is a schematic drawing showing a state during clipping work in the body cavity;
FIG. 4C is a schematic drawing showing a state in which the capsule-indwelling-type medical device is placed in the body cavity;
FIG. 5A is a schematic perspective view showing a configuration example of a latching portion of a first modification;
FIG. 5B is a schematic perspective view showing another configuration example of the latching portion of the first modification;
FIG. 5C is a schematic perspective view showing still another configuration example of the latching portion of the first modification;
FIG. 5D is a schematic perspective view showing still another configuration example of the latching portion of the first modification;
FIG. 5E is a schematic perspective view showing still another configuration example of the latching portion of the first modification;
FIG. 6 is a sectional construction drawing showing a configuration example of a capsule-indwelling-type medical device according to a second embodiment of the invention;
FIG. 7 is an explanatory view for explaining a function of a hood portion;
FIG. 8 is a sectional construction drawing showing a configuration example of a capsule-indwelling-type medical device of a second modification;
FIG. 9 is a sectional construction drawing showing a configuration example of a capsule-indwelling-type medical device of a third modification;
FIG. 10 is a sectional construction drawing showing a configuration example of a capsule-indwelling-type medical device of a fourth modification;
FIG. 11 is a sectional construction drawing showing a configuration example of a capsule-indwelling-type medical device of a fifth modification;
FIG. 12 is a sectional construction drawing showing a configuration example of a capsule-indwelling-type medical device according to a third embodiment of the invention;
FIG. 13 is a perspective view showing a schematic configuration example of a capsule-indwelling-type medical device of a sixth modification;
FIG. 14 is a perspective view showing a modification example of the sixth modification;
FIG. 15 is a schematic perspective view showing a configuration example of a capsule-indwelling-type medical device according to a fourth embodiment of the invention;
FIG. 16 is a sectional construction drawing showing a configuration example of a capsule-indwelling-type medical device;
FIG. 17 is a schematic drawing showing a configuration example of a wireless capsule-indwelling-type medical device;
FIG. 18 is a sectional view showing a state in which the capsule-indwelling-type medical device is retained at a front edge of the endoscope;
FIG. 19A is a schematic drawing showing a state in which the endoscope is introduced into the body cavity;
FIG. 19B is a schematic drawing showing a state in which the capsule-indwelling-type medical device is released in the body cavity from the endoscope;
FIG. 19C is schematic drawing showing a state in which the capsule-indwelling-type medical device is latched;
FIG. 20 is a schematic perspective view showing a state in which the capsule-indwelling-type medical device is latched to a body cavity tissue;
FIG. 21 is a schematic plan view showing a placed and fixed state;
FIG. 22 is a schematic sectional view showing a placed and fixed state;
FIG. 23A is a schematic perspective view showing a configuration example of a seventh modification;
FIG. 23B is a schematic perspective view showing a state in which the capsule-indwelling-type medical device is captured and removed;
FIG. 24 is a schematic perspective view showing a configuration example of an eighth modification;
FIG. 25 is a schematic perspective view showing a configuration example of a ninth modification;
FIG. 26 is a schematic front view showing a latched state of the ninth embodiment;
FIG. 27 is a schematic front view showing the latched state of the capsule-indwelling-type medical device when an orientation of the latching member is changed;
FIG. 28 is a schematic perspective view showing a configuration example of a tenth modification;
FIG. 29 is a schematic perspective view showing an example in which the plural latching members are provided;
FIG. 30 is a schematic perspective view showing a configuration example of an eleventh modification;
FIG. 31 is a schematic perspective view showing a state in which a mesh member is folded;
FIG. 32 is a schematic perspective view showing a configuration example in which a sheet-like member is used;
FIG. 33 is a schematic perspective view showing a configuration example of a fifth embodiment of the invention;
FIG. 34A is a schematic perspective view showing a state in which one side of the capsule-indwelling-type medical device is latched;
FIG. 34B is a schematic perspective view showing a state in which both sides of the capsule-indwelling-type medical device are latched;
FIG. 35 is a schematic plan view showing a configuration example of a twelfth modification;
FIG. 36 is a schematic perspective view showing a configuration example of a thirteenth modification;
FIG. 37 is a schematic front view showing a configuration example of a fourteenth modification;
FIG. 38 is a schematic side view showing a configuration example of a sixth embodiment of the invention; and
FIG. 39 is a schematic perspective view showing a configuration example of a fifteenth modification.

### EXPLANATIONS OF LETTERS OR NUMERALS

- 100: CAPSULE-INDWELLING-TYPE MEDICAL DEVICE
- 200: INDWELLING DEVICE FOR AN ENCAPSULATED MEDICAL DEVICE
- 201: LATCHING PORTION
- 202: RETAINING UNIT
- 203: CLIP
- 204: STRING MEMBER
- 210 and 210a: HOLE PORTIONS
- 220: CLIP
- 221: BODY CAVITY TISSUE
- 222: ANNULAR STRING MEMBER
- 223: MESH MEMBER
- 230: NEEDLE
- 231: SUCTION PORTION
- 240: RETAINING UNIT
- 241: HOOD PORTION
- 242: ABUTTING PORTION
- 250: RETAINING UNIT
- 251: HOOD PORTION
- 260: RETAINING UNIT
- 261: HOOD PORTION
- 262: ABUTTING PORTION
- 263: DIRT PROTECTIVE COATING FILM
- 264: HOOD PORTION WITH OPTICAL CHARACTERISTICS
- 270: RETAINING UNIT
- 271: HOOD PORTION
- 300: CAPSULE ENDOSCOPE
- 307: OBSERVATION OPTICAL SYSTEM
- 400: SUBJECT
- 410: ENDOSCOPE HEMOSTATIC CLIP
- 411: BODY CAVITY TISSUE
- 500: INDWELLING DEVICE FOR AN ENCAPSULATED MEDICAL DEVICE
- 501: RETAINING MEMBER
- 502: LATCHING MEMBER
- 503: LATCHING PORTION
- 504: SETTING PORTION
- 505: LATCHING MEMBER
- 506: LATCHING PORTION
- 510: LATCHING MEMBER
- 511: LATCHING PORTION
- 515: LATCHING MEMBER
- 520: LATCHING MEMBER
- 521: LATCHING PORTION
- 525: LATCHING MEMBER
- 526: LATCHING PORTION
- 527: JOINT PORTION
- 530: LATCHING MEMBER
- 535: LATCHING PORTION
- 536: LATCHING MEMBER
- 541 and 542: LATCHING PORTIONS
- 543 and 544: LATCHING MEMBERS
- 550 and 551: LATCHING PORTIONS
- 552: LATCHING MEMBER
- 555: LATCHING PORTION
- 556: LATCHING MEMBER
- 557: JOINT PORTION
- 558: BANDING MEMBER
- 560: LATCHING PORTION
- 561: LATCHING MEMBER
- 565: LATCHING MEMBER
- 566: LATCHING PORTION
- 600: CAPSULE-INDWELLING-TYPE MEDICAL DEVICE
- O: OBSERVATION AXIS
- L: GENERATRIX

### BEST MODE FOR CARRYING OUT THE INVENTION

An exemplary embodiments of an indwelling device for an encapsulated medical device, an in vivo indwelling device for a capsule endoscope, and a capsule-indwelling-type medical device according to the invention will be described below with reference to the accompanying drawings. The invention is not limited to the exemplary embodiments. In the drawings, the same component or the relevant component is designated by the same reference numeral.

### (First embodiment)

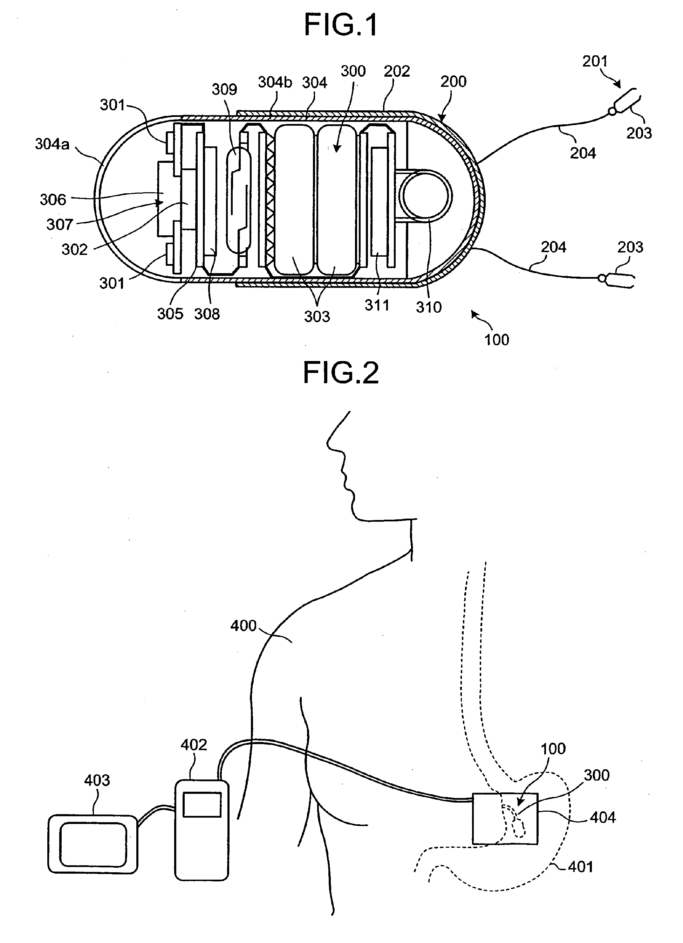

A first embodiment of the invention will be described. FIG. 1 is a sectional construction drawing showing a configuration example of a capsule-indwelling-type medical device. A capsule-indwelling-type medical device 100 includes an indwelling device for an encapsulated medical device 200 and a capsule endoscope 300. The capsule endoscope 300 is an encapsulated medical device mounted to and retained in the indwelling device for an encapsulated medical device 200.

Basically the capsule endoscope 300 is an already-existing and general-purpose capsule endoscope for alimentary canal. The capsule endoscope 300 is swallowed from the oral cavity and introduced into the body cavity of the subject, the capsule endoscope 300 takes the image in the body cavity as the information in body cavity, and the capsule endoscope 300 wirelessly transmits and outputs the data of the taken image in the body cavity.

The capsule endoscope 300 will be described with reference to FIG. 1. The capsule endoscope 300 includes plural illumination portions 301, an image pickup device 302, a button-shape battery 303, and a capsule type chassis 304. The illumination portions 301 formed by LED illuminate the body cavity of the subject. The image pickup device 302 formed by CCD, CMOS, or the like takes the image of the body cavity. The button-shape battery 303 supplies electric power to the illumination portion 301 and the image pickup device 302. The illumination portion 301, the image pickup device 302, and the battery 303 are arranged in the capsule type chassis 304. A silver oxide battery, a storage battery, a power generation battery, and the like can be used as the battery 303.

The capsule type chassis 304 includes a transparent, hemispherical dome-shape front-edge cover chassis 304a and a cylindrical body chassis 304b, and the capsule type chassis 304 is formed in a size which can be swallowed from the oral cavity of the subject. The front-edge cover chassis 304a covers the illumination portion 301 and the like. The front-edge cover chassis 304a and the capsule type chassis 304 are provided in a water-tight manner, and the battery 303 and the like are arranged in the cylindrical body chassis 304b. The body chassis 304b is made of a colored material through which visible light is not transmitted.

The image pickup device 302 is mounted on an imaging board 305, and an optical system 306 including an imaging lens is arranged in front of the image pickup device 302. An observation optical system 307 includes the illumination portion 301, the image pickup device 302, and the optical system 306. A controller 308 which controls each unit is mounted on the backside of the imaging board 305.

The capsule endoscope 300 includes a reed switch 309 therein. The reed switch 309 is turned on and off according to an external magnetic field in order to control drive of the capsule endoscope 300. In the storage state of the capsule endoscope 300, the capsule endoscope 300 is stored in a package including a permanent magnet which supplies the external magnetic field. The off state is kept in the capsule endoscope 300 in an environment where the magnetic field having at least predetermined strength is applied, and the capsule endoscope 300 is turned on by decreasing the external magnetic field. Therefore, the capsule endoscope 300 is not driven in the state in which the capsule endoscope 300 is stored in the package.

The capsule endoscope 300 includes a transmitter 311 with an antenna 310 on the backside of the battery 303. The transmitter 311 wirelessly transmits the information on the taken image to the outside of the subject.

On the other hand, the indwelling device for an encapsulated medical device 200 includes a latching portion 201 and a retaining unit 202. The latching portion 201 fixes and places the capsule endoscope 300 to the body cavity tissue located at the desired region. The latching portion 201 is provided in the retaining unit 202, and the retaining unit 202 mounts and retains the capsule endoscope 300. The latching portion 201 of the first embodiment includes an endoscope hemostatic clip 203 which is directly latched in and fixed to the body cavity tissue by the endoscopic clipping treatment tool, and the clip 203 is coupled to the retaining unit 202 by a short string member 204. In the first embodiment, each two of the clips 203 and the string members 204 are provided while coupled to the different positions of the retaining unit 202.

The retaining unit 202 has a structure in which the retaining unit 202 integrally retains the capsule endoscope 300 while being in surface contact with a peripheral surface of the capsule endoscope 300, so that the retaining unit 202 integrally retains the fitted and mounted capsule endoscope 300. Specifically, the retaining unit 202 is formed in a shape including a substantially cylindrical portion having substantially the same radius as the capsule endoscope 300, and the retaining unit 202 is also formed in a cap shape which wholly covers the capsule endoscope 300 except for a portion on the side of the front-edge cover chassis 304a.

Examples of a method in which the capsule endoscope 300 is mounted to and retained in the retaining unit 202 may include a method of press-fitting the capsule endoscope 300, a method in which the capsule endoscope 300 is temporarily attached with a heat-shrinkable tube or the like and then the capsule endoscope 300 is securely retained by heating and shrinking the tube, and a method of fixing and retaining the capsule endoscope 300 with a bonding agent or the like. That is, the already-existing capsule endoscope 300 can be mounted, the retaining state in which the mounted capsule endoscope 300 does not drop off easily from the retaining unit 202 can be maintained during a period in which the monitoring is performed in the body cavity, and a swallowing property (introduction-into-body-cavity property) similar to that of the capsule endoscope 300 can be secured. The retaining unit 202 may be made of a material which does not lose the observation function and transmission function of the capsule endoscope 300 and does not have a harmful influence on a living body even if the material is introduced and placed in the body cavity. Either a hard member or a soft member may be used as the retaining unit 202, and either a transparent member or an opaque member may be used as the retaining unit 202. Unlike the capsule endoscope 300, there is a small restriction in coupling the string member 204 to the retaining unit 202 which does not includes contents, so that the string member 204 can easily and firmly be coupled to the retaining unit 202.

The capsule-indwelling-type medical device 100 including the capsule endoscope 300 mounted to and retained in the indwelling device for an encapsulated medical device 200 is combined with the receiver and the like in the state in which the capsule-indwelling-type medical device 100 is placed and fixed to the desired region in a subject 400, which allows the capsule-indwelling-type medical system to be configured. FIG. 2 is a schematic drawing showing a configuration example of a wireless capsule-indwelling-type medical system. As shown in FIG. 2, the wireless capsule-indwelling-type medical system includes the capsule-indwelling-type medical device 100, a portable receiver 402, and a display device 403. The capsule-indwelling-type medical device 100 includes the capsule endoscope 300. The capsule endoscope 300 is introduced into the subject 400, the capsule endoscope 300 is placed in and fixed to the desired region in the body cavity such as a stomach 401 to take the color image in the stomach 401, and the capsule endoscope 300 wirelessly transmits the image signal data to the receiver 402. The receiver 402 receives the color image data wirelessly transmitted from the capsule endoscope 300. The display device 403 such as a portable viewer displays the color image based on the video signal received by the receiver 402. The receiver 402 includes a receiving antenna 404 which adheres to the region corresponding to the point at which the capsule endoscope 300 is placed and fixed, e.g., the neighborhood of the stomach 401 in the outer surface of the subject 400.

Therefore, although the general-purpose capsule endoscope 300 for alimentary canal is used, the capsule endoscope 300 is placed in and fixed to the desired region in the body cavity as the capsule-indwelling-type medical device 100, the image of the desired region is taken by the capsule endoscope 300, the taken image in the body cavity is observed by the display device 403, and thereby the capsule-indwelling-type medical device 100 is suitably used to monitor a diseased site after the operation.

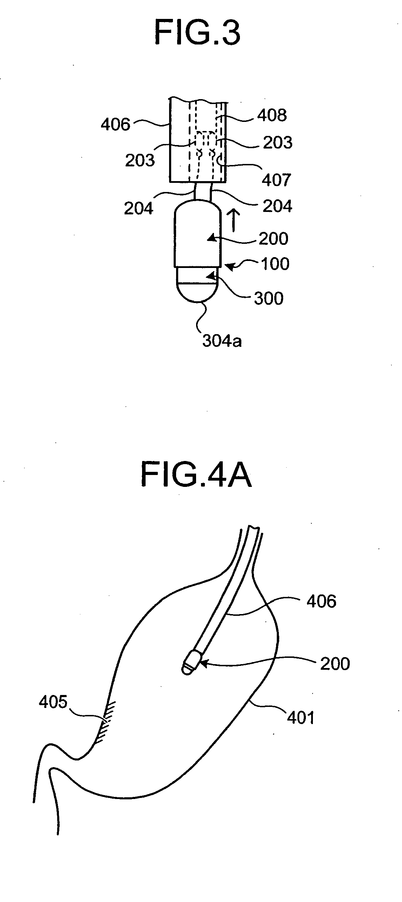

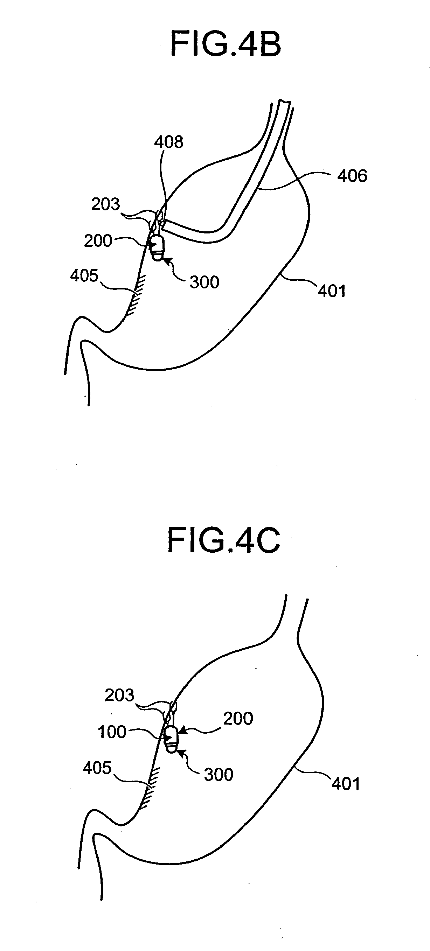

A procedure of medical action including the working of placing the capsule endoscope 300 in the body cavity will be described with reference to FIGS. 3 and 4. The introduction and placement of the capsule endoscope 300 in the body cavity are performed to monitor the presence or absence of the bleeding in the diseased site after the endoscopic operation, and the introduction and placement of the capsule endoscope 300 are performed after the endoscopic operation of the target subject 400. Alternatively, the capsule endoscope 300 may be introduced and placed in the body cavity prior to the endoscopic operation, and the placement working is not performed after the endoscopic operation. In the drawings including FIG. 4, the reference numeral 405 denotes a region (for example, mucosa removal region) to be operated by the endoscopic operation. It is assumed that the adhesion of the receiving antenna 404 to the outside surface of the subject 400 is performed at appropriate timing around the time when the capsule endoscope 300 is introduced into the body cavity.

As shown in FIG. 3, the general-purpose capsule endoscope 300 for alimentary canal is prepared, and the capsule endoscope 300 is fitted in and mounted to the retaining unit 202, which allows the capsule endoscope 300 to be integral with the retaining unit 202. The clip 203 coupled to the retaining unit 202 is assembled in a clipping treatment tool 408 which is of the endoscopic treatment tool projected from a forceps channel 407 of an endoscope 406. Both the clip 203 and the clipping treatment tool 408 are brought in the forceps channel 407 to temporarily fix the capsule endoscope 300 to the front edge portion of the endoscope 406 along with the retaining unit 202.

As shown in FIG. 4A, while the capsule endoscope 300 and the retaining unit 202 are integral with each other, the endoscope 406 is introduced into the body cavity through the oral cavity of the subject 400. After the endoscope 406 is introduced into the desired region, e.g., the stomach 401, the clipping treatment tool 408 is projected again to separate the capsule endoscope 300 retained by the retaining unit 202 from the front edge of the endoscope 406.

As shown in FIG. 4B, the clip 203 is latched in and fixed to the body cavity tissue by the clipping treatment tool 408, while the confirmation whether or not the region to be operated 405 which is of the monitoring target is located within the observation visual field of the observation optical system 307 of the capsule endoscope 300 is made by monitoring the image taken by the capsule endoscope 300 through the receiver 402 and the display device 403. At this point, in the first embodiment, the two clips 203 are prepared, and the image taken by the capsule endoscope 300 is monitored through the display device 403 to adjust the latched point of one of the clips 203 after the other clip 203 is latched in the body cavity. Therefore, a placement attitude can be adjusted in the capsule endoscope 300.

Then, the endoscope 406 is extracted from the body cavity, and the capsule endoscope 300 is placed in and fixed to the desired region in the body cavity through the retaining unit 202 and clip 203 which attach and retain the capsule endoscope 300 as shown in FIG. 4C, which allows the indwelling device observation to be performed with the capsule endoscope 300.

After the monitoring is performed, the clip 203 drops off in the body cavity along with the retaining unit 202 and capsule endoscope 300 due to necrosis of the body cavity tissue of the portion where the clip 203 is latched, so that the dropped-off capsule endoscope 300 which is integral with the retaining unit 202 may directly be captured in an endoscopic manner with a capturing net or the like, or the capsule endoscope 300 may be discharged to the outside of the body cavity.

In the first embodiment, the capsule endoscope 300 is introduced and placed from the oral cavity by way of example. The capsule endoscope 300 can similarly be applied in the case where the capsule endoscope 300 is introduced and placed from not only the oral cavity but also other openings, e.g., an anus.

### (First modification)

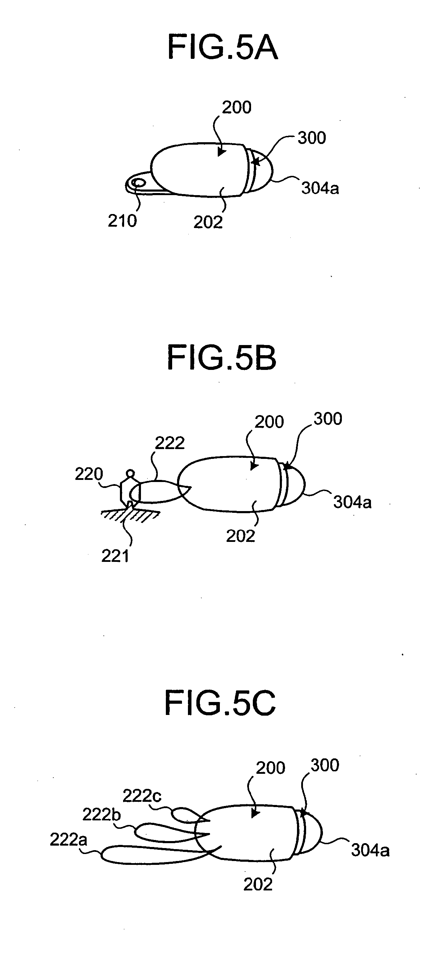

FIGS. 5A to 5E illustrate modification examples of the latching portion. FIG. 5A shows a modification, in which a hole portion 210 is formed as the latching portion. The hole portion 210 is integral with the retaining unit 202 while a part of the retaining unit 202 is projected and the hole portion 210, and the endoscopic treatment tool fixes the hole portion 210 to the body cavity tissue using an endoscopic fixture, e.g., an endoscope hemostatic clip (not shown). According to the modification of FIG. 5A, the structure is simplified and the latching portion is easily produced.

FIG. 5B shows a modification in which an annular string member 222 is formed as the latching portion. The endoscopic treatment tool such as the grasping forceps fixes the annular string member 222 to a body cavity tissue 221 using the endoscopic fixture such as an endoscope hemostatic clip 220, which is provided while coupled to the retaining unit 202. According to the modification of FIG. 5B, the annular string member 222 can simply be coupled in a ring shape, so that the structure is simplified and the latching portion is easily produced.

In this case, for example as shown in FIG. 5C, when plural annular string members 222a to 222c which are of the latching portion are provided while being different from one another in the size, the optimum one of the annular string members 222a to 222c can be selected according to the shape of the body cavity tissue 221 near the desired region in latching the latching portion in the body cavity tissue 221 with the clip 220, so that an approach to the desired region is easily made to improve the workability in the endoscopic treatment.

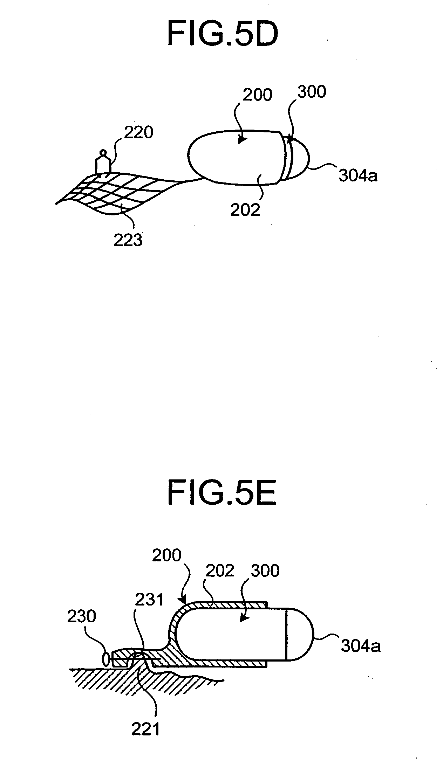

FIG. 5D shows a modification in which a mesh member 223 is formed as the latching portion. The endoscopic treatment tool such as the grasping forceps fixes the mesh member 223 to the body cavity tissue 221 using the endoscopic fixture such as the endoscope hemostatic clip 220, which is provided while coupled to the retaining unit 202. According to the modification of FIG. 5C, the mesh member 223 can be latched in the body cavity tissue 221 at an arbitrary position, so that the capsule endoscope 300 is easily latch and fixed by the clip 220 to improve the workability in the endoscopic treatment. The same effect is obtained in the case where a fabric member or a sheet-shape member is used in place of the mesh member 223.

FIG. 5E shows a modification in which a suction unit 231 is formed as the latching portion. The suction portion 231 is integral with the retaining unit 202 while a part of the retaining unit 202 is projected and the hole portion 210, and the suction portion 231 sucks a part of the body cavity tissue 221 to be fixed by, e.g., a needle 230.

### (Second embodiment)

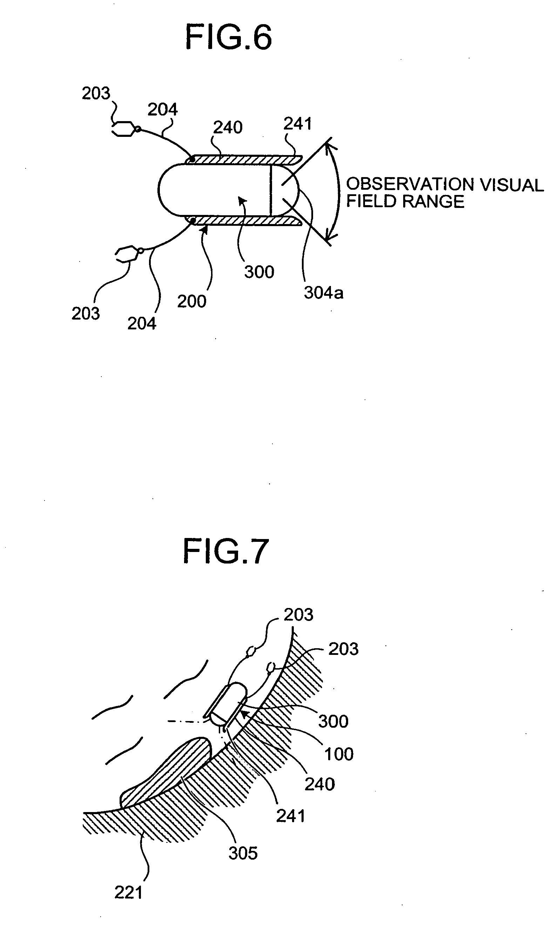

A second embodiment of the invention will be described with reference to FIGS. 6 and 7. FIG. 6 is a sectional construction drawing showing a configuration example of a capsule-indwelling-type medical device of the second embodiment. The indwelling device for an encapsulated medical device 200 of the second embodiment includes a retaining unit 240 having substantially the same radius as the capsule endoscope 300. The retaining unit 240 is formed in the substantially cylindrical shape while both ends of the retaining unit 240 are opened. The front edge on the side of the front-edge cover chassis 304a of the retaining unit 240 is formed as a hood portion 241 which is projected for the observation optical system 307 incorporated in the capsule endoscope 300. The projection amount of hood portion 241 is set such that the hood portion 241 does not enter the observation visual field of the observation optical system 307, i.e., the hood portion 241 is located outside the observation visual field. Although the above-described configurations can be used as the latching portion, the same configuration as FIG. 1 in which the clip 203 is used is adopted in the second embodiment.

The capsule endoscope 300 takes the image in the body cavity through the transparent front-edge cover chassis 304a using the observation optical system 307. Therefore, the proper image cannot be obtained when dirt or the like is generated in the front-edge cover chassis 304a which becomes the observation window. In the case where the indwelling device observation whose purpose is to monitor the diseased site after the operation, because meat and drink are not generally taken in during the observation period, it is thought that the dirt is slightly generated on the surface of the front-edge cover chassis 304a due to the meat and drink. However, because the peristaltic movement is performed in the body cavity, there is a possibility of adhesion of a liquid such as a bodily fluid to the surface of the front-edge cover chassis 304a during the long-term observation. In the first embodiment, because the retaining unit 240 includes the hood portion 241, the dirt can be prevented in the surface of the front-edge cover chassis 304a while the capsule endoscope 300 is placed in the body cavity, and the observation visual field of the observation optical system 307 is not interrupted even if the retaining unit 240 is made of the opaque material. Therefore, the good indwelling device observation can be performed by the observation optical system 307.

Particularly, as shown in FIG. 7, when the capsule endoscope 300 is placed in the stomach 401 so as to perform the downward observation with respect to the gravity direction, because the retaining unit 240 which integrally retains the capsule endoscope 300 includes the hood portion 241 on the front edge side of the retaining unit 240, the bodily fluid and the like hardly adheres to the surface of the front-edge cover chassis 304a which becomes the observation window, which allows the dirt to be prevented in the front-edge cover chassis 304a.

### (Second modification)

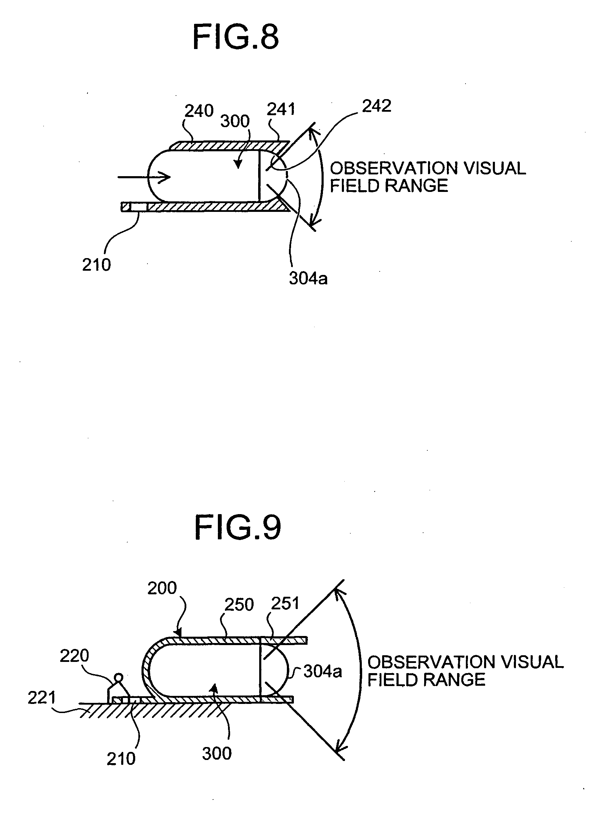

FIG. 8 is a sectional construction drawing showing a configuration example of a capsule-indwelling-type medical device of a second modification. The hood portion 241 of the second modification includes an abutting portion 242 for the capsule endoscope 300. In the abutting portion 242, an inner peripheral portion on the front edge side is projected toward a radial direction such that the inner peripheral portion mates with the outer surface shape of the front-edge cover chassis 304a of the capsule endoscope 300. That is, because an inner diameter of the abutting portion 242 is smaller than an outer diameter of the capsule endoscope 300, the capsule endoscope 300 is stopped at the position where the capsule endoscope 300 abuts on the abutting portion 242 of the hood portion 241 when the capsule endoscope 300 is fitted in and mounted to the retaining unit 240 from the direction show by an arrow of FIG. 8. Therefore, because it is not necessary to perform alignment between the retaining unit 240 and the capsule endoscope 300, the fitting and attachment working becomes simplified.

### (Third modification)

FIG. 9 is a sectional construction drawing showing a configuration example of a capsule-indwelling-type medical device of a third modification. A retaining unit 250 of the third modification includes a hood portion 251 formed by a transparent member. The whole of the retaining unit 250 may be formed by the transparent member, or only the hood portion 251 may be formed by the transparent material. The hood portion 251 is formed while projected to the position where the hood portion 251 enters the observation visual field of the observation optical system 307. Specifically, the hood portion 251 is formed while projected from the front edge portion of the front-edge cover chassis 304a of the capsule endoscope 300, and an upper portion of the hood portion 251 is formed longer than a lower portion of the hood portion 251 in the gravity direction.

According to the third modification, the hood portion 251 is formed sufficiently long and the hood portion 251 covers the surface of the front-edge cover chassis 304a to a large extent. Therefore, the bodily fluid and the like hardly adhere to the front-edge cover chassis 304a and the dirt protection effect can be enhanced. In this case, the length of the hood portion 251 is ensured by causing the hood portion 251 to enter the observation visual field. However, the hood portion 251 is formed by the transparent member, so that the observation ability can be secured through the observation optical system 307.

### (Fourth modification)

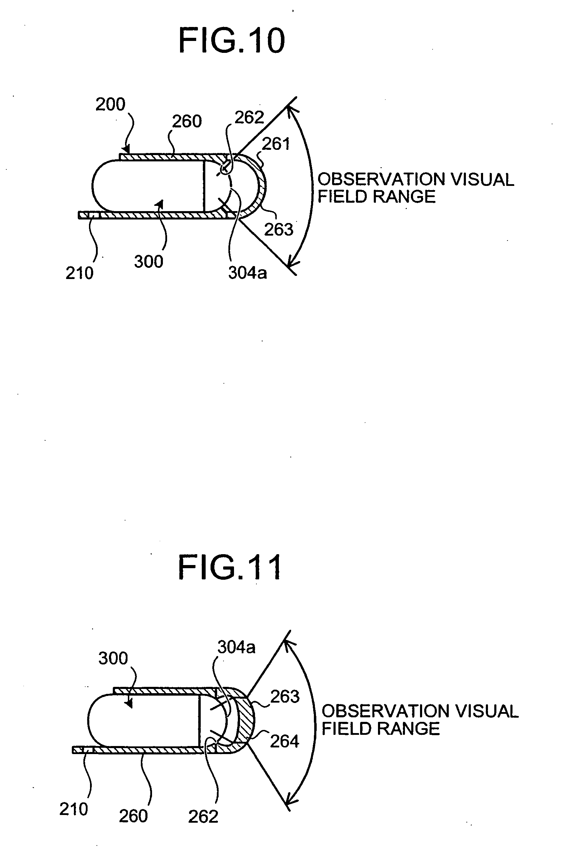

FIG. 10 is a sectional construction drawing showing a configuration example of a capsule-indwelling-type medical device of a fourth modification. A retaining unit 260 of the fourth modification includes a dome-shape hood portion 261 formed by the transparent member. The hood portion 261 covers the whole of the observation visual field of the observation optical system 307. The whole of the retaining unit 260 may be formed by the transparent member, or only the hood portion 261 may be formed by the transparent material. In the retaining unit 260, an abutting portion 262 corresponding to the abutting portion 242 is formed while located at a boundary between the retaining unit 260 and the hood portion 261. A dirt protective coating is applied to the outer surface of the hood portion 261 to form a dirt protective coating film 263. The dirt protective coating may be a water-repellent coating or a hydrophilic coating in which any photocatalyst is used. Any dirt protective coating may be used for a specific application (according to the purpose for using an indwelling device, the indwelling site, and the like).

According to the fourth modification, the hood portion 261 covers the whole of the observation visual field (the whole surface in front of the front-edge cover chassis 304a), so that the dirt is not generated in the surface of the front-edge cover chassis 304a of the capsule endoscope 300. Because the dirt protective coating film 263 which is of the dirt protective coating is formed on the outer surface of the hood portion 261, the dirt is hardly generated in the hood portion 261, which has a small adverse influence on the observation optical system 307. Particularly, when the surface position of the hood portion 261 is set so as to be located outside a focal depth of the observation optical system 307 of the capsule endoscope 300, the dirt is not brought into focus in the observation optical system 307 even if the dirt is slightly generated in the outer surface of the hood portion 261, so that the influence on the observation can be decreased as much as possible.

The dirt protective coating can also be applied to the outer surface of the front-edge cover chassis 304a of the capsule endoscope 300. However, because it is necessary to select the water-repellent coating or the hydrophilic coating according to the region where the capsule endoscope 300 is used, versatility is lost in the capsule endoscope 300. On the other hand, when the dirt protective coating is applied to the outer surface of the hood portion 261, even if the general-purpose capsule endoscope 300 is used, it is possible to select the water-repellent coating or the hydrophilic coating, so that option of the material becomes widened.

### (Fifth modification)

FIG. 11 is a sectional construction drawing showing a configuration example of a capsule-indwelling-type medical device of a fifth modification. In the configuration of the fourth embodiment, the retaining unit 260 of the fifth modification includes a dome-shape hood portion 264 having the optical characteristics which acts on the observation optical system 307. In the configuration of the fifth modification, the optical characteristics such as a refractive index and a curvature of the material of the hood portion 264 and a curvature are appropriately adjusted to change the angle of view or the depth of field in the image taken by the observation optical system 307. In the example shown in FIG. 11, the hood portion 264 is configured to have the optical characteristics in which an observation view angle is widened by adjusting the angle of view. According to the fifth modification, the optical performance can be obtained according to the purpose for placing the capsule endoscope 300, and the monitoring observation can be performed better.

### (Third embodiment)

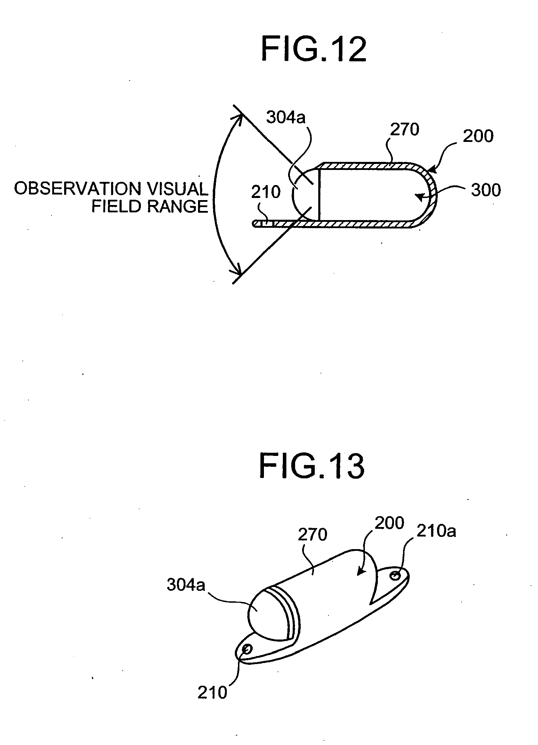

A third embodiment of the invention will be described with reference to FIG. 12. FIG. 12 is a sectional construction drawing showing a configuration example of a capsule-indwelling-type medical device of the third embodiment. In configuration of the indwelling device for an encapsulated medical device 200 of the third embodiment, the latching portion which is integral with a retaining unit 270, e.g., the hole portion 210 is formed while located on the side of the front-edge cover chassis 304a of the capsule endoscope 300, and the hole portion 210 enters the observation visual field of the observation optical system 307.

According to the third embodiment, because the hole portion 210 also enters the observation visual field, the latched state of the hole portion 210 can be confirmed by seeing the screen of the display device 403 during the working of latching and fixing the clip or the like in and to the hole portion 210, and the clip can be securely latched in and fixed to the hole portion 210. In the monitoring after the capsule endoscope 300 is placed and fixed, not only the target region to be operated but also the state of the hole portion 210 can be monitored, so that generation of the drop-off can instantaneously be found in the hole portion 210.

### (Sixth modification)

FIG. 13 is a perspective view showing a schematic configuration example of the capsule-indwelling-type medical device 100 of a sixth modification. The sixth modification is one in which, in the retaining unit 270 having the configuration of the third embodiment, a hole portion 210a which is of the latching portion is also made on the rear portion side of the capsule endoscope 300 (at the back of the observation visual field) while located at the same side (on the same generatrix) as the hole portion 210. The neighborhoods of the hole portions 210 and 210a are formed by a member having flexibility in order to easily introduce the capsule endoscope 300 into the body cavity.

According to the sixth modification, the retaining unit 270 in which the capsule endoscope 300 is mounted and retained is latched in and fixed to the body cavity tissue at the two hole portions 210 and 210a in the lengthwise direction. Therefore, the fixed state becomes secure, and the visual field can securely be fixed in the capsule endoscope 300. Particularly, after the hole portion 210a is latched, when the latching working is performed on the side the hole portion 210 while the image taken by the capsule endoscope 300 is monitored by the display device 403, the visual filed of the capsule endoscope 300 can securely be fixed with respect to the desired region.

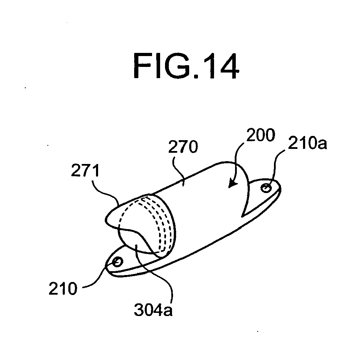

In this case, as shown in FIG. 14, a transparent hood portion 271 which can be separated from the retaining unit 270 may detachably be provided in a retro-fitted manner in a portion of the retaining unit 270 on the side of the front-edge cover chassis 304a of the capsule endoscope 300. This enables the hood portion 271 to be attached and detached according to the application or the region to be monitored.

### (Fourth embodiment)

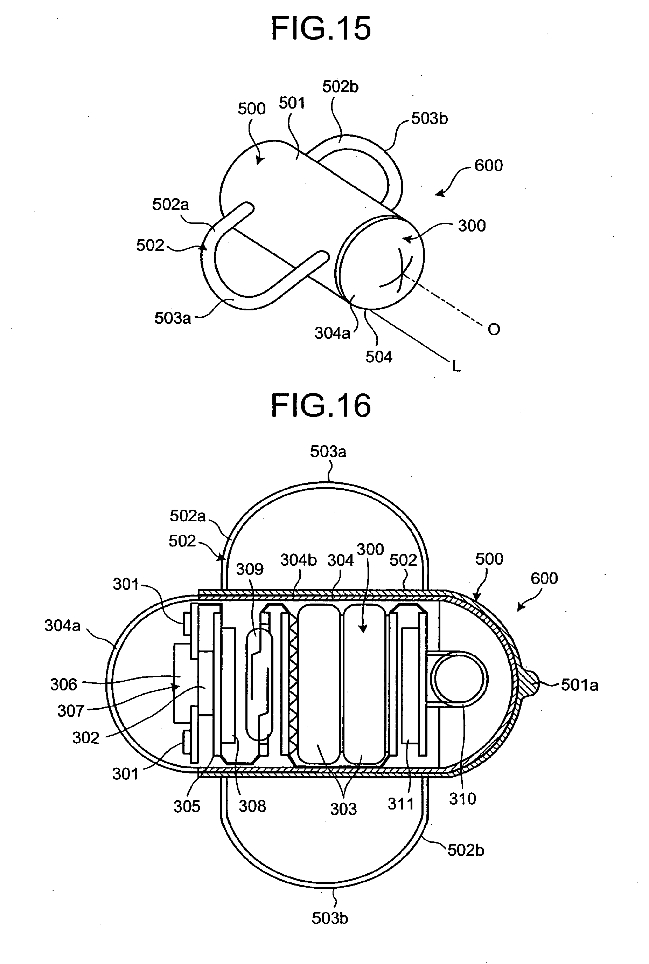

A fourth embodiment of the invention will be described below. FIG. 15 is a schematic perspective view showing a configuration example of a capsule-indwelling-type medical device of the fourth embodiment, and FIG. 16 is a sectional construction drawing showing a configuration example of the capsule-indwelling-type medical device. A capsule-indwelling-type medical device 600 includes an indwelling device for an encapsulated medical device 500 and the capsule endoscope 300. The capsule endoscope 300 is the encapsulated medical device mounted to and retained in the indwelling device for an encapsulated medical device 500. For example, FIG. 16 shows the configuration of the capsule endoscope 300, and the configuration of the capsule endoscope 300 is similar to that described in the first embodiment.

On the other hand, the indwelling device for an encapsulated medical device 500 includes a retaining member 501 and a latching member 502. The retaining member 501 mounts and retains the capsule endoscope 300. The latching member 502 is provided while coupled to the retaining member 501, and the latching member 502 latches and fixes the capsule endoscope 300 in and to the body cavity tissue in the desired region.

The retaining member 501 has a structure in which the retaining member 501 integrally retains the capsule endoscope 300 while being in surface contact with the peripheral surface of the capsule endoscope 300, so that the retaining member 501 integrally retains the fitted and mounted capsule endoscope 300. Specifically, the retaining member 501 is formed in the shape including the substantially cylindrical portion having substantially the same radius as the capsule endoscope 300, and the retaining member 501 is also formed in the cap shape which wholly covers the capsule endoscope 300 except for a portion on the side of the front-edge cover chassis 304a. The retaining member 501 includes a projected grip portion 501a at an end portion thereof, and the grip portion 501a is grasped by the later-mentioned grasping forceps.

Examples of a method in which the capsule endoscope 300 is mounted to and retained in the retaining member 501 may include a method of press-fitting the capsule endoscope 300, a method in which the capsule endoscope 300 is temporarily attached with a heat-shrinkable tube or the like and then the capsule endoscope 300 is securely retained by heating and shrinking the tube, and a method of fixing and retaining the capsule endoscope 300 with a bonding agent or the like. That is, the already-existing capsule endoscope 300 can be mounted, the retaining state in which the mounted capsule endoscope 300 does not drop off easily from the retaining member 501 can be maintained during a period in which the monitoring is performed in the body cavity, and a swallowing property (introduction-into-body-cavity property) similar to that of the capsule endoscope 300 can be secured. The retaining unit 501 may be made of a material which does not lose the observation function and transmission function of the capsule endoscope 300 and does not have a harmful influence on a living body even if the material is introduced and placed in the body cavity. Either a hard member or a soft member may be used as the retaining member 501, and either a transparent member or an opaque member may be used as the retaining member 501.

The latching members 502 are integrally formed in a grip shape (substantially U-shape having joint portions to the retaining member 501 at positions separated from each other in an observation axis direction) while projected on both sides of the retaining member 501 such that the latching members 502 are symmetrical in relation to an observation axis O in a plane including the observation axis O. A front edge portion of the latching member 502 is formed as a latching portion 503. That is, the pair of latching members 502 includes two latching portions 503a and 503b at positions which are located outside projection plane of the capsule endoscope to the body cavity tissue, the position sandwiching the capsule endoscope 300. Specifically, the latching members 502 include the latching portions 503a and 503b latched in the body cavity tissue at the positions where the latching members 502 sandwich the observation axis O of the observation optical system 307.

From the different angle, in the case where a generatrix L in a lowermost portion of the retaining member 501 (capsule endoscope 300) is set to a setting portion 504 placed in the body cavity tissue, the latching member 502 of the fourth embodiment includes a first latching member 502a and a second latching member 502b. The first latching member 502a has a first latching portion 503a attached to the body cavity tissue by the later-mentioned endoscopic fixture while provided at the position upwardly separated from the setting portion 504 with respect to the retaining member 501 (capsule endoscope 300). The second latching member 502b has a second latching portion 503b which is provided at the position shifted from the first latching portion 503a while provided at the position upwardly separated from the setting portion 504 with respect to the retaining member 501 (capsule endoscope 300), and the second latching portion 503b is attached to the body cavity tissue by the later-mentioned endoscopic fixture. As used herein, "lowermost portion" or "upward direction" shall not mean a vertical direction. In the positional relationship between the indwelling device for an encapsulated medical device 500 and the body cavity tissue, "lowermost portion" or "downward direction" shall mean the position or direction closer to the body cavity tissue of the retaining member 501 (capsule endoscope 300), and "uppermost portion" or "upward direction" shall mean the position or direction separated farther away from the body cavity tissue. The latching members 502a and 502b and the latching portions 503a and 503b are appropriately expressed and explained in the form of the latching member 502 and the latching portion 503 if needed. For the purpose of simple explanation, discrimination with suffixes a and b is neglected, and the same holds for a pair of latching members and a pair of latching portions in the following embodiments and modifications.

The latching portions 503a and 503b are latched in the body cavity tissue by the endoscopic fixture, i.e., an endoscope hemostatic clips 410 in the fourth embodiment. The latching member 502 is made of at least an elastic material. The latching member 502 can be folded around the retaining member 501 (i.e., around the capsule endoscope 300) in introducing the indwelling device for an encapsulated medical device 500 into the body cavity, and the latching member 502 can recover and expand in the originally fixed grip shape by releasing the latching member 502 at the desired position in the body cavity. Unlike the capsule endoscope 300, there is a small restriction in adding the latching member 502 to the retaining unit 501 which does not includes contents, so that the latching member 502 can easily be realized. It is not always necessary that the latching member 502 be integral with the retaining member 501. The latching member 502 may be separated from the retaining member 501. That is, it is necessary that the latching member 502 and the retaining member 501 be integrally coupled.

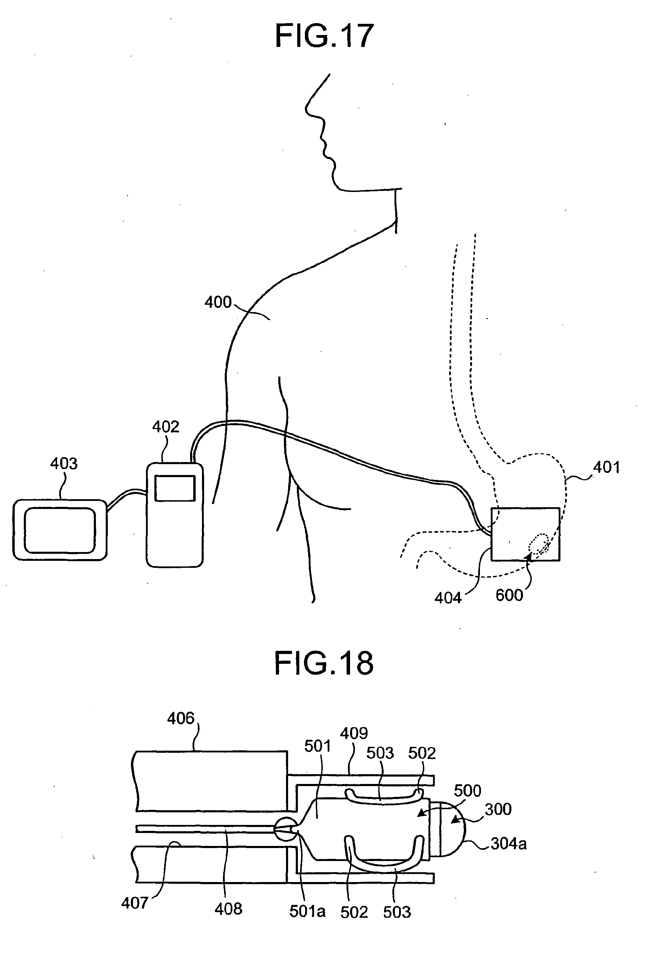

The capsule-indwelling-type medical device 600 including the capsule endoscope 300 mounted to and retained in the indwelling device for an encapsulated medical device 500 is combined with the receiver and the like in the state in which the capsule-indwelling-type medical device 600 is placed in and fixed to the desired region in the subject 400, which allows the capsule-indwelling-type medical system to be configured. FIG. 17 is a schematic drawing showing a configuration example of a wireless capsule-indwelling-type medical device. As shown in FIG. 17, the wireless capsule-indwelling-type medical device includes the capsule-indwelling-type medical device 600, the portable receiver 402, and the display device 403. The capsule-indwelling-type medical device 600 includes the capsule endoscope 300. The capsule endoscope 300 is introduced into the subject 400, the capsule endoscope 300 is placed in and fixed to the desired region in the body cavity such as the stomach 401 to take the color image in the stomach 401, and the capsule endoscope 300 wirelessly transmits the image signal data to the receiver 402. The receiver 402 receives the color image data wirelessly transmitted from the capsule endoscope 300. The display device 403 such as the portable viewer displays the color image based on the video signal received by the receiver 402. The receiver 402 includes the receiving antenna 404 which adheres to the region corresponding to the point at which the capsule endoscope 300 is placed and fixed, e.g., the neighborhood of the stomach 401 in the outer surface of the subject 400.

Therefore, although the general-purpose capsule endoscope 300 for alimentary canal is used, the capsule endoscope 300 is placed in and fixed to the desired region in the body cavity as the capsule-indwelling-type medical device 600, the image of the desired region is taken by the capsule endoscope 300, the taken image in the body cavity is observed by the display device 403, and thereby the capsule-indwelling-type medical device 100 is suitably used to monitor the diseased site after the operation.

A procedure of placing the capsule endoscope 300 in the body cavity will be described with reference to FIGS. 18 to 20. The introduction and placement of the capsule endoscope 300 in the body cavity are performed to monitor the presence or absence of the bleeding in the diseased site after the endoscopic operation, and the introduction and placement of the capsule endoscope 300 are performed after the endoscopic operation of the target subject 400. Alternatively, the capsule endoscope 300 may be introduced and placed in the body cavity prior to the endoscopic operation, and the placement working is not performed after the endoscopic operation. It is assumed that the adhesion of the receiving antenna 404 to the outside surface of the subject 400 is performed at appropriate timing around the time when the capsule endoscope 300 is introduced into the body cavity.

The general-purpose capsule endoscope 300 for alimentary canal is prepared, and the capsule endoscope 300 is fitted in and mounted to the retaining member 501, which allows the capsule endoscope 300 to be integral with the retaining member 501. As shown in FIG. 18, a grasping portion 501a located at an end portion of the retaining member 501 is grasped by the grasping forceps 408 passing through the forceps channel 407 of the endoscope 406, and the capsule-indwelling-type medical device 600 is brought in a cylindrical conveyance member 409 at the front edge of the endoscope 406. At this point, the latching member 502 is curved and folded so as to be wound around the retaining member 501, and the latching member 502 is brought in the conveyance member 409 such that an excessive projected portion is not generated around the conveyance member 409.

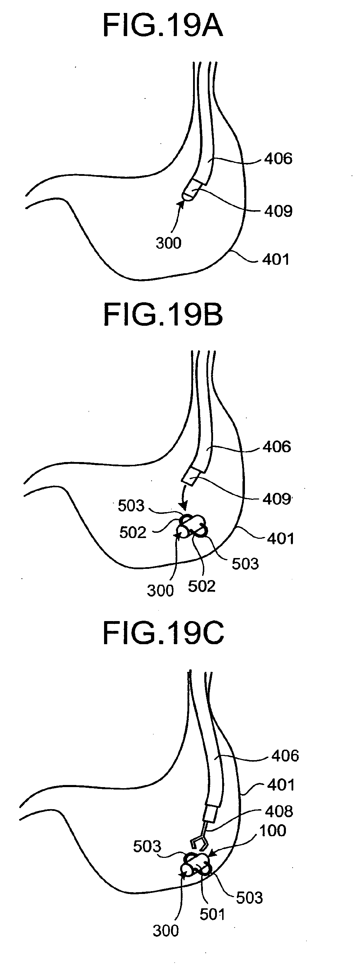

As shown in FIG. 19A, the front edge side of the endoscope 406 is introduced into the body cavity while the capsule-indwelling-type medical device 600 is brought in the conveyance member 409. At this point, because the latching member 502 and the like are not projected, the latching member 502 and the like do not interrupt the introduction of the endoscope 406 into the body cavity, and the endoscope 406 can be introduced like the single body of the capsule endoscope 300.

When the front edge side of the endoscope 406 is introduced to the desired region in the body cavity, e.g., the neighbor of monitoring point after the operation in the stomach 401, the capsule-indwelling-type medical device 600 is released from the conveyance member 409 into the body cavity by releasing the grasping forceps 408 as shown in FIG. 19B. In the capsule-indwelling-type medical device 600, the folded latching member 502 recovers and expands to the grip shape which is of the originally fixed shape by elastic restoring force in association with the releasing operation in the body cavity.

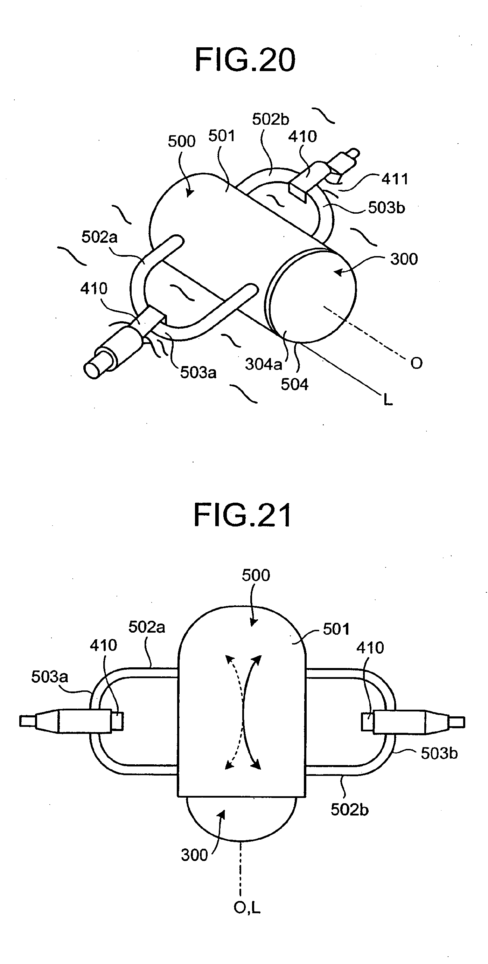

Then, as shown in FIGS. 19B and 20, the endoscopic treatment is performed to the capsule-indwelling-type medical device 600 released in the body cavity using the endoscopic treatment tool such as the grasping forceps 408, which allows the two latching portions 503a and 503b at the front edges of the latching members 502 to be latched in and fixed to the body cavity tissue 411 by the two endoscopic fixtures, i.e., the two endoscope hemostatic clips 410 in the fourth embodiment. At this point, the latching member 502 is slightly pulled outward so as to pull retaining member 501 with tension, and the latching portions 503a and 503b are latched in and fixed to the body cavity tissue 411 by the endoscope hemostatic clips 410.

At this point, the latching portions 503a and 503b are latched in and fixed to the body cavity tissue 411 by operating endoscope hemostatic clips 410 with the grasping forceps 408 or the like, while the confirmation whether or not the region to be operated which is of the monitoring target is located within the observation visual field of the observation optical system 307 of the capsule endoscope 300 is made by monitoring the image taken by the capsule endoscope 300 through the receiver 402 and the display device 403. In this case, in the fourth embodiment, the two latching portions 503a and 503b are prepared, and the image taken by the capsule endoscope 300 is monitored through the display device 403 to adjust the point where one of the latching portion 503b (or 503a) is latched by the endoscope hemostatic clip 410 after the other latching portion is latched in the body cavity by the endoscope hemostatic clip 410. Therefore, the indwelling attitude can be adjusted in the capsule endoscope 300.

Then, the endoscope 406 is extracted from the body cavity, and the capsule endoscope 300 is placed in and fixed to the desired region in the body cavity through the retaining member 501 and latching member 502 which attach and retain the capsule endoscope 300 as shown in FIG. 20, which allows the indwelling device observation to be performed with the capsule endoscope 300.

After the monitoring is performed, the endoscope hemostatic clips 410 drop off in the body cavity along with the retaining member 501 and capsule endoscope 300 due to the necrosis of the body cavity tissue of the portion where the endoscope hemostatic clip 410 is latched, so that the dropped-off capsule endoscope 300 which is integral with the retaining member 501 may directly be captured in an endoscopic manner with the capturing net or the like, or the capsule endoscope 300 may be discharged to the outside of the body cavity.

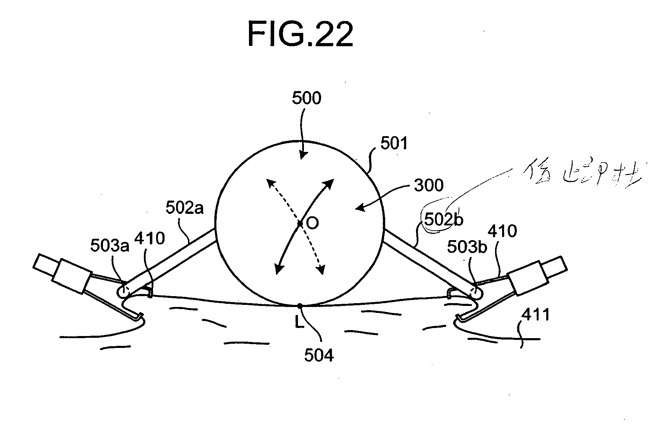

The placed and fixed state of the capsule endoscope 300 (retaining member 501) will be described with reference to FIGS. 21 and 22. FIG. 21 is a schematic plan view showing the placed and fixed state, and FIG. 22 is a schematic sectional view showing the placed and fixed state. In the fourth embodiment, the two latching portions 503a and 503b of the latching member 502 are latched in the body cavity tissue 411 with the tension by the endoscope hemostatic clips 410 at the positions located outside the projection plane of the capsule endoscope to the body cavity tissue 411, and the capsule endoscope 300 is clamped at the positions in the direction orthogonal to the observation axis O. Therefore, there is obtained the placed and fixed state where the generatrix L of the capsule endoscope 300 which is parallel to the observation axis O comes stably into line contact with the body cavity tissue 411 in the desired direction, and the placed state in which the orientation of the observation visual field of the observation optical system 307 or the observation axis O is not shifted can be secured.

That is, in the fourth embodiment, the latching member 502 is used to latch the two latching portions 503a and 503b in the body cavity tissue 411 with the tension by the endoscope hemostatic clips 410. The latching member 502 includes the first latching member 502a and the second latching member 502b. The first latching member 502a has the first latching portion 503a which is provided at the position upwardly separated from the setting portion 504 with respect to the retaining member 501 (capsule endoscope 300). The second latching member 502b has the second latching portion 503b which is provided at the position shifted from the first latching portion 503a while provided at the position upwardly separated from the setting portion 504 with respect to the retaining member 501 (capsule endoscope 300). Therefore, there is obtained the placed and fixed state where the setting portion 504 of the retaining member 501(capsule endoscope 300) which is parallel to the observation axis O comes stably into contact with the body cavity tissue 411 in the desired direction, and the placed state in which the orientation of the observation visual field of the observation optical system 307 or the observation axis O is not shifted can be secured.

Particularly, even if the capsule endoscope 300 with the indwelling device for an encapsulated medical device 500 is viewed from the top as shown in FIG. 21, or even if the capsule endoscope 300 with the indwelling device for an encapsulated medical device 500 is viewed from the side as shown in FIG. 22, the positions where the indwelling device for an encapsulated medical device 500 is latched by the latching portions 503a and 503b are set such that the moment shown by the arrow acting on the capsule endoscope 300 about the latched position by the latching portion 503a is canceled by the moment about the latching portion 503b, and the latching portions 503a and 503b are latched in the body cavity tissue 411 with the tension by the endoscope hemostatic clips 410. Therefore, the body cavity can continuously and stably be monitored without generating the swing by which the orientation of the observation visual field is shaken.

### (Seventh modification)

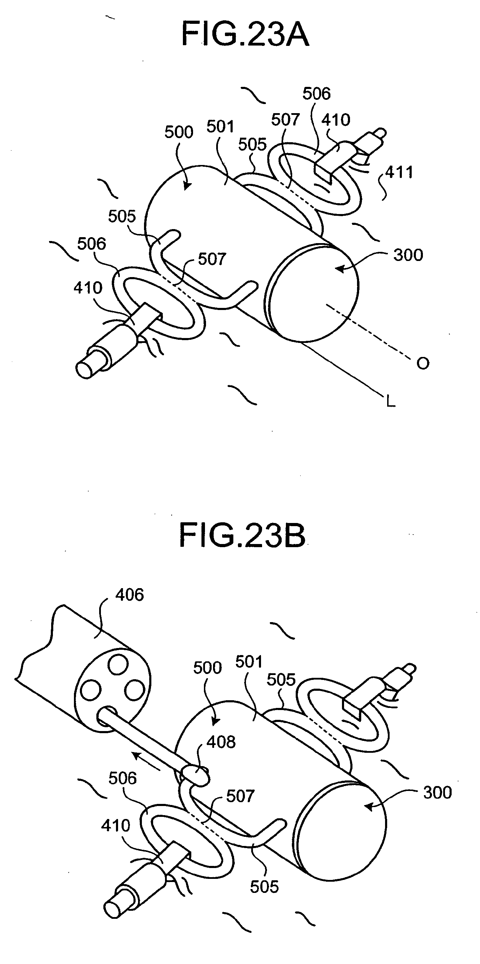

A seventh modification will be described with reference to FIGS. 23A and 23B. FIG. 23A is a schematic perspective view showing a configuration example of the seventh modification, and FIG. 23B is a schematic perspective view showing a state in which the capsule-indwelling-type medical device is captured and removed. In the seventh modification, as shown in FIG. 23A, a latching member 505 which is formed in a substantially 8-shape or ∝ shape when viewed from the top is used in place of the latching member 502, a latching portion 506 is formed in a outside front-edge portion, and a cut line 507 which is easily cut is made at a boundary portion.

According to the seventh embodiment, after the monitoring operation, as shown in FIG. 23B, the endoscope 406 is introduced to grasp and pull the inside portion of the latching member 505 with the grasping forceps 408 or the like, and the latching member 505 is cut at the cut line 507. Therefore, the capsule endoscope 300 can be captured while the latching portion 506 is left by the endoscope hemostatic clip 410.

### (Eighth modification)

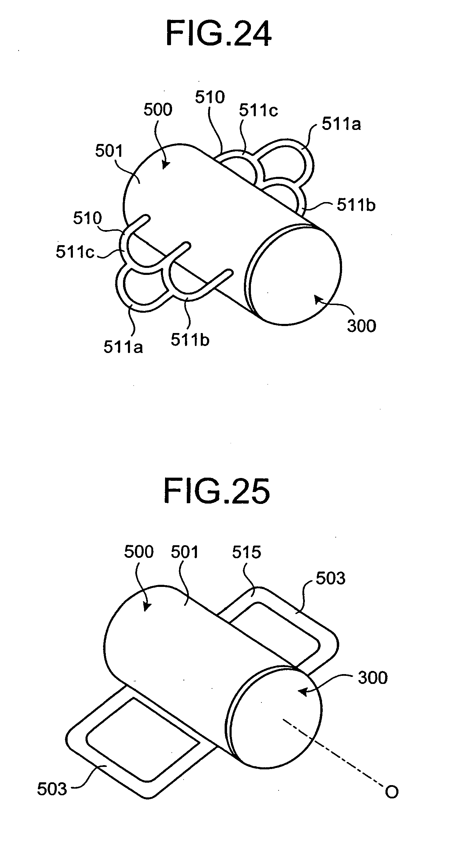

An eighth modification will be described with reference to FIG. 24. FIG. 24 is a schematic perspective view showing a configuration example of the eighth modification. In the eighth modification, instead of the latching member 502 having the simple grip shape, latching members 510 located on both sides of the retaining member 501 are formed in the shape having the multi stages of plural latching portions 511a, 511b, 511c, ... by overlapping the grip shape in the multi stages. According to the configuration of the eighth embodiment, in the working of latching the indwelling device for an encapsulated medical device 500 in the body cavity tissue 411 with the endoscope hemostatic clip 410, the latching working can be performed by selecting the point which is easily clipped in the plural one-side latching portions 511a, 511b, 511c, ... according to the status of the body cavity tissue 411 in the latched region, so that the working can easily be performed by the endoscopic treatment.

### (Ninth modification)

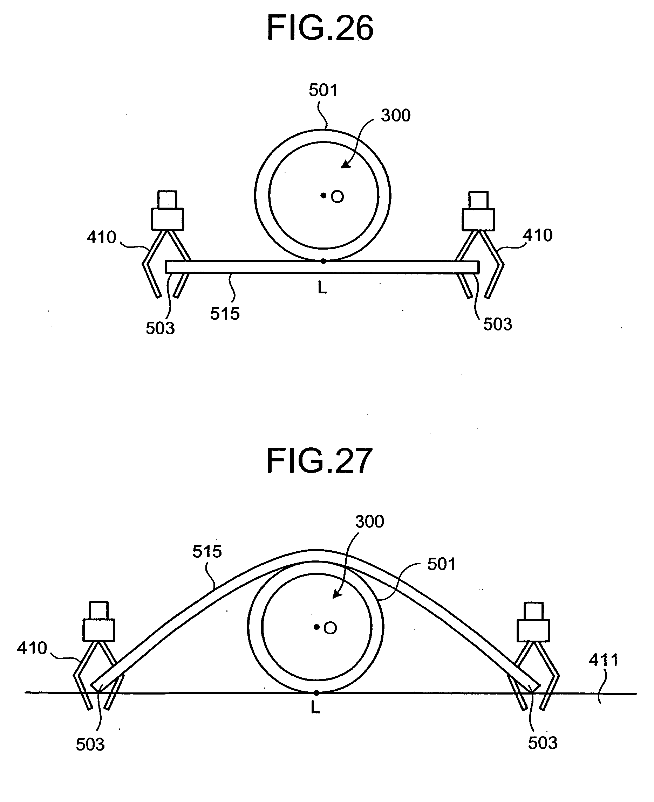

A ninth modification will be described with reference to FIGS. 25 and 26. FIG. 25 is a schematic perspective view showing a configuration example of the ninth modification, and FIG. 26 is a schematic front view showing a latched state of the ninth modification. In the ninth modification, an integrally formed latching member 515 is provided in place of the latching members 502 provided on both sides of the retaining member 501 in the plane including the observation axis O. The latching member 515 is biased on one side of the retaining member 501 (capsule endoscope 300) in a tangential manner. That is, the latching member 515 is formed in the continuously plane shape on the both sides of the retaining member 501.

According to the configuration of the ninth embodiment, in releasing the capsule-indwelling-type medical device 600 into the body cavity from the conveyance member 409 described in FIG. 19B, the latching member 515 having the continuous plane shape is in surface contact with the body cavity tissue 411 as shown in FIG. 26. Therefore, the indwelling device for an encapsulated medical device 500 is hardly turned over, and the stable state is easily adopted in the indwelling device for an encapsulated medical device 500 compared with the case where the cylindrical retaining member 501 is in direct contact with the body cavity tissue 411, so that the latching working of the latching portion 503 with the endoscope hemostatic clip 410 becomes easier and more secure.

In the configuration shown in FIG. 25, as shown in FIG. 27, a joint portion between the retaining member 501 and the latching member 515 is arranged on the side opposite to the body cavity tissue 411 to be latched, and the latching portion 503 may be latched in the body cavity tissue 411 by the endoscope hemostatic clip 410. According to the configuration shown in FIG. 27, as shown by the arrow in FIG. 27, force for pressing the capsule endoscope 300 against the body cavity tissue 411 is strongly applied by the latching member 515 latched with the tension, so that the generatrix L can come securely into line contact with the body cavity tissue 411.

### (Tenth modification)

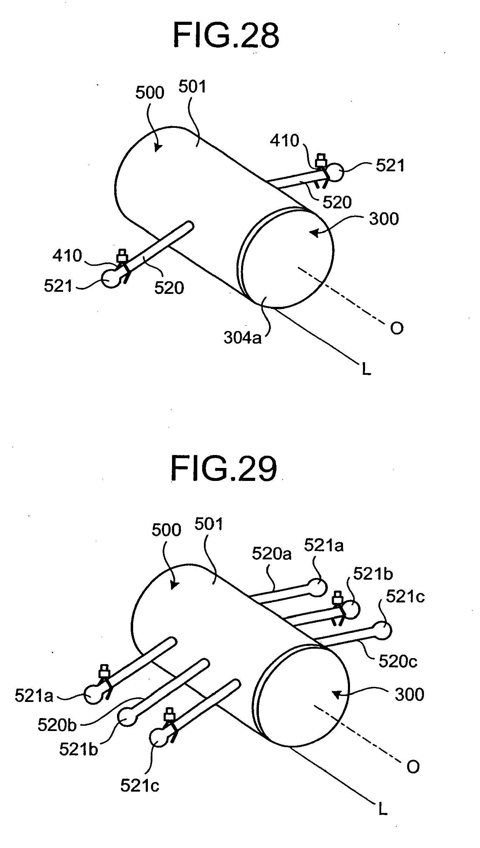

A tenth modification will be described with reference to FIG. 28. FIG. 28 is a schematic perspective view showing a configuration example of the tenth modification. In the tenth modification, a rod-shape latching member 520 is provided in place of the grip-shape latching member 502 while being integral with the retaining member 501. The latching member 520 has a latching portion 521 having a larger diameter. The latching members 520 are formed in the direction orthogonal to the observation axis O, and the latching portions 521 are arranged at the position where the latching portion 521 sandwiches the observation axis O. As with the latching member 502, the latching member 520 is made of the elastic material. The elastic material can be folded around the retaining member 501 (capsule endoscope 300) in introducing the indwelling device for an encapsulated medical device 500 into the body cavity, and the elastic material can recover and expand to the rod shape which is of the originally fixed shape in releasing the indwelling device for an encapsulated medical device 500 in the body cavity. The latching member 520 is integral with the retaining member 501 on the plane including the observation axis O. Alternatively, as with the case described in FIG. 25 and the like, the latching member 520 may be coupled so as to come into line contact with the retaining member 501 on one side of the retaining member 501.

The same effect can be obtained by the configuration of the tenth embodiment. The number of rod-shape latching members 520 is not limited to one. For example, plural latching members 520a to 220c having latching portions 521a to 221c may be provided as shown in FIG. 29. According to the configuration shown in FIG. 29, as with the case shown in FIG. 24, in the working of latching the indwelling device for an encapsulated medical device 500 in the body cavity tissue 411 with the endoscope hemostatic clip 410, the latching working can be performed by selecting the point where the latching portion is easily clipped in the plural latching portions 521a to 221c according to the state of the body cavity tissue 411 which is of the latched region, and the latching working is easily performed by the endoscopic treatment.

### (Eleventh modification)

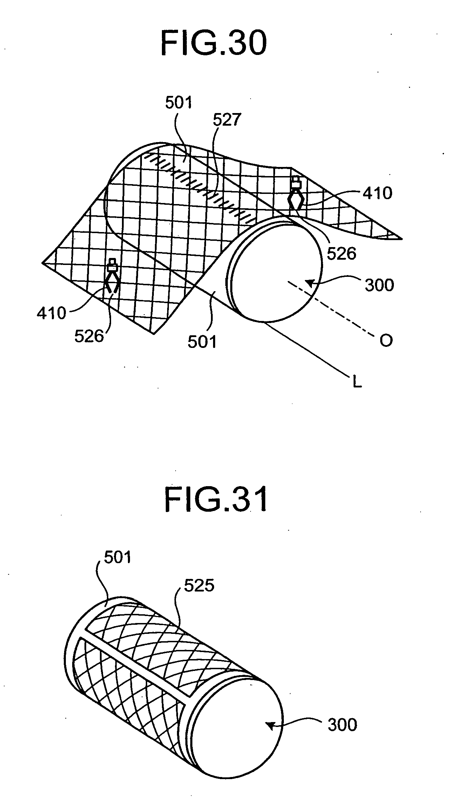

An eleventh modification will be described with reference to FIGS. 30 and 31. FIG. 30 is a schematic perspective view showing a configuration example of the eleventh modification, and FIG. 31 is a schematic perspective view showing a state in which a mesh member is folded. In the eleventh modification, instead of the latching members 502 and 520 which have the latching portions 503 and 521 at the particular points respectively, a latching member 525 formed by a mesh member which has a latching portion 526 at an arbitrary point is provided in the direction orthogonal to the observation axis O by bonding the latching member 525 to the retaining member 501 at a joint portion 527 with a bonding agent or the like. The latching member 525 has a width corresponding to a length of the retaining member 501, and the joint portion 527 is set over the whole width of the retaining member 501 in the axis direction on the outer surface of the retaining member 501. As shown in FIG. 31, the latching member 525 is made of an elastic material. The elastic material can be folded so as to be wounded around the retaining member 501, and the elastic material can recovered and expand to the original net shape in the released state.

In the configuration of the eleventh embodiment, the latching member 525 is introduced into the body cavity while folded as shown in FIG. 31. When the indwelling device for an encapsulated medical device 500 is released in the body cavity from the conveyance member 409, the indwelling device for an encapsulated medical device 500 becomes the released state to cause the latching member 525 to recover and expand to the expanding net shape. Therefore, as shown in FIG. 30, the latching member 525 is arranged on the upper side of the retaining member 501 (capsule endoscope 300), and the observation visual field of the observation optical system 307 is adjusted so as to be orientated toward the desired direction. Then, the latching portion 526 is latched in the body cavity tissue 411 by the endoscope hemostatic clip 410 while the tension is applied to the latching member 525 by the retaining member 501. At this point, the latching member 525 is formed by the mesh member, and the latching member 525 may be latched at any position by the endoscope hemostatic clip 410. That is, because the position of the latching portion 226 can be arbitrarily selected, latching can be done by selecting the position where the latching is easily done , and the latching workability is improved. On each side of the retaining member 501 (capsule endoscope 300), the latching can be arbitrarily done at plural points.

Even in latching the latching member 525 by the mesh member, the latching member 525 is latched in the body cavity tissue 411 with the tension at positions located outside the projection plane of the capsule endoscope to the body cavity tissue 411, the latching portions 226 clamping the capsule endoscope 300 at the positions. Therefore, because the orientation of the joint portion 527 is fixed, the generatrix L of the retaining member 501 (capsule endoscope 300) comes into line contact with the body cavity tissue 411 in the desired direction, and the latching can be performed such that the orientation or the observation axis O of the observation visual field is not shifted. Particularly, as shown in FIG. 30, when the joint portion 527 is arranged at the position opposite to the body cavity tissue 411 to latch the latching member 525, like the case shown in FIG. 27, the force for pressing the capsule endoscope 300 against the side of the body cavity tissue 411 can also be applied, and the stable latched state is realized. Like the case shown in FIG. 26, the joint portion 227 may be arranged at the position on the side of the body cavity tissue 411 to latch the latching member 525.

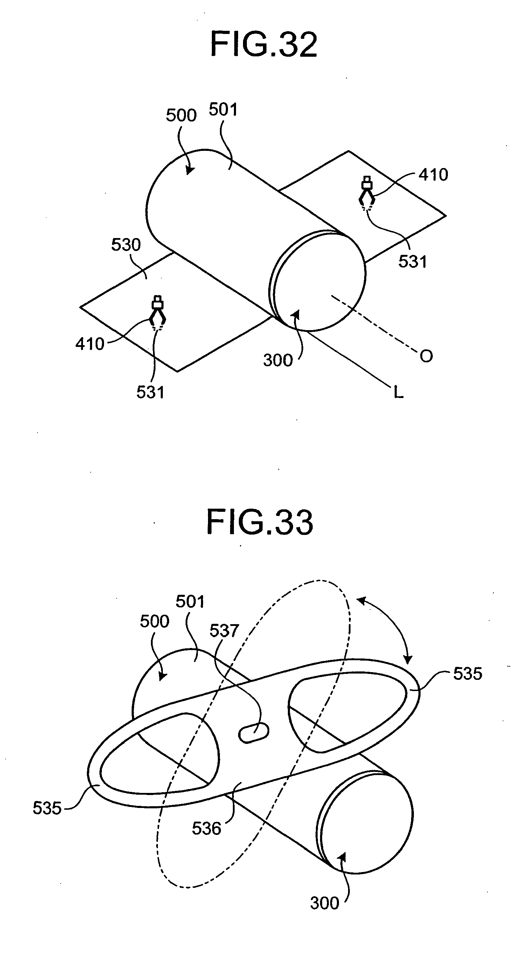

As shown in FIG. 32, when a latching member 530 formed by a fabric sheet-shape member is used in place of the latching member 525 formed by the mesh member, the same effect can be obtained. The reference numeral 531 denotes an arbitrary latching portion which is specified by the position where a hole is made by inserting and latching the endoscope hemostatic clip 410. The latching member 530 and the retaining member 501 are integrally coupled by the joint portion (not shown) similar to the joint portion 527.

### (Fifth embodiment)

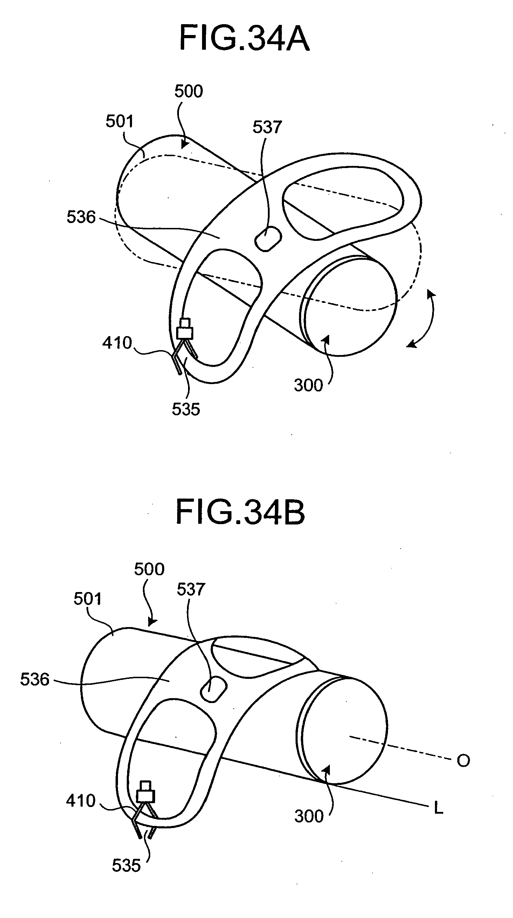

A fifth embodiment of the invention will be described with reference to FIGS. 33 to 34B. FIG. 33 is a schematic perspective view showing a configuration example of the fifth embodiment of the invention, FIG. 34A is a schematic perspective view showing a state in which one side of the capsule-indwelling-type medical device is latched, and FIG. 34B is a schematic perspective view showing a state in which both sides of the capsule-indwelling-type medical device are latched. In the fifth embodiment, a latching member 536 is formed long and thin while having latching portions 535 on both sides thereof, and the latching member 536 and the retaining member 501 are provided while being able to be mutually rotated by coupling the latching member 536 onto the peripheral surface of the retaining member 501 by a rotating fulcrum 537. The latching member 536 is made of an elastic material. The elastic material can be folded around the retaining member 501 (accordingly, around the capsule endoscope 300) in introducing the capsule endoscope 300 into the body cavity, and the elastic material can recover and expand to the rod shape which is of the originally fixed shape in releasing the capsule endoscope 300 in the body cavity.

In the above configuration, after the capsule-indwelling-type medical device 600 is introduced into and released at the desired position in the body cavity, the placement position and visual field direction of the capsule endoscope 300 are roughly determined while the latching member 536 is arranged such that the rotating fulcrum 237 is located on the side opposite to the body cavity tissue 411, and the latching portion 535 on one side is latched in the body cavity tissue 411 by the endoscope hemostatic clip 410 as shown in FIG. 34A. In this state of things, as shown by a phantom line in FIG. 34A, the placement position and visual field direction of the capsule endoscope 300 are finely adjusted while the display device 403 is monitored. In the state where the desired placement position and visual field direction are obtained, as shown in FIG. 34B, the remaining latching portion 535 of the latching member 536 is slightly pulled outward, and the latching portion 535 is latched in the body cavity tissue 411 by the endoscope hemostatic clip 410 while the tension is applied to the latching portion 535 by the retaining member 501. Therefore, the force for pressing the retaining member 501 (capsule endoscope 300) against the side of the body cavity tissue 411 is also applied by the latching member 536, the retaining member 501 (capsule endoscope 300) is not rotated with respect to the latching member 236, and the state in which the retaining member 501 comes into line contact with the body cavity tissue 411 is maintained while the generatrix L is kept in the desired direction, so that the visual field can correctly be obtained.

When the external force is required by the endoscopic treatment tool in the rotation between the retaining member 501 and the latching member 536, the latching member 536 may be arranged and latched such that the rotating fulcrum 537 is located on the side of the body cavity tissue 41.

### (Twelfth modification)

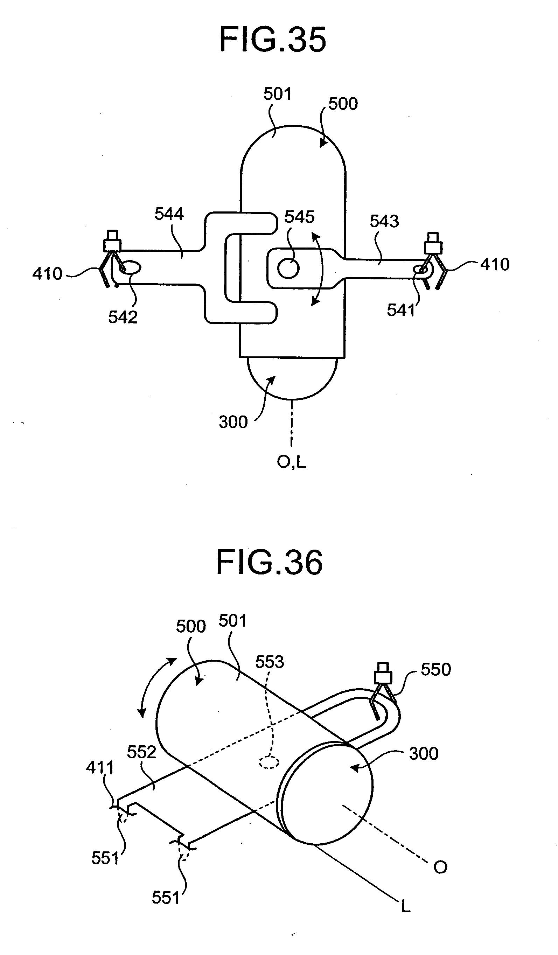

A twelfth modification will be described with reference to FIG. 35. FIG. 35 is a schematic plan view showing a configuration example of the twelfth modification. The twelfth modification includes two latching members 543 and 544 in place of the latching member 536. The latching members 543 and 544 include latching portions 541 and 542 at end portions thereof respectively. The latching member 543 is rotatably coupled to the peripheral surface of the retaining member 501 about a rotating fulcrum 545, and the latching member 544 is fixedly coupled to the peripheral surface of the retaining member 501 while extended toward the side opposite to the latching member 543 with respect to the retaining member 501. Particularly, the joint portion of the latching member 544 at the retaining member 501 is formed in a bifurcate shape having two joint units which are separated from each other in a lengthwise direction of the retaining member 501. The latching members 543 and 544 are made of an elastic material. The elastic material can be folded around the retaining member 501 (accordingly, around the capsule endoscope 300) in introducing the capsule endoscope 300 into the body cavity, and the elastic material can recover and expand to the rod shape which is of the originally fixed shape in releasing the capsule endoscope 300 in the body cavity.

In the above configuration, after the capsule-indwelling-type medical device 600 is introduced into and released at the desired position in the body cavity, the placement position and visual field direction of the capsule endoscope 300 are roughly determined while the latching members 543 and 544 are arranged such that the rotating fulcrum 544 and the joint portion are located on the side opposite to the body cavity tissue 411. Then, the latching portion 541 on the movable side is latched in the body cavity tissue 411 by the endoscope hemostatic clip 410. In this state of things, because the retaining member 501 is rotatable with respect to the latching member 543, the placement position and visual field direction of the capsule endoscope 300 are finely adjusted while the display device 403 is monitored. In the state where the desired placement position and visual field direction are obtained, the remaining latching portion 542 of the latching member 544 on the fixed side is slightly pulled outward, and the latching portion 542 is latched in the body cavity tissue 411 by the endoscope hemostatic clip 410 while the tension is applied to the latching portion 542 by the retaining member 544. Therefore, the retaining member 501 is not moved with respect to the latching member 544, and the force for pressing the retaining member 501 (capsule endoscope 300) against the side of the body cavity tissue 411 is also applied by the latching members 543 and 544. As a result, the retaining member 501 (capsule endoscope 300) is not rotated with respect to the latching member 236, the state in which the retaining member 501 comes into line contact with the body cavity tissue 411 is maintained while the generatrix L is kept in the desired direction, and the visual field can correctly be obtained.

### (Thirteenth modification)

A thirteenth modification will be described with reference to FIG. 36. FIG. 36 is a schematic perspective view showing a configuration example of the thirteenth modification. In the thirteenth modification, a latching member 552 is coupled to the peripheral surface of the retaining member 501 by a rotating fulcrum 553, which allows the latching member 552 and the retaining member 501 to be mutually rotated. The latching member 552 is formed long and thin while having latching portions 550 and 551 at both ends thereof. The forced external force is required for the mutual rotation between the latching member 552 and the retaining member 501 using the endoscopic treatment tool such as the endoscope and forceps, and the semi-fixed coupling structure is formed between the latching member 552 and the retaining member 501 such that the latching member 552 and the retaining member 501 are not mutually rotated.

As with the cases of the above embodiments and modifications, the latching portion 550 is formed as a portion which is latched in the body cavity tissue 411 by the endoscope hemostatic clip 410, and the latching portion 551 is formed as the latching portion which is directly latched in the body cavity tissue 411 by the endoscopic treatment because the latching portion 551 has the function of latching itself in the body cavity tissue 411. In the thirteenth modification, the two latching portion 551 are formed in the outward spike shape. Alternatively, the

The latching member 552 is made of an elastic material. The elastic material can be folded around the retaining member 501 (accordingly, around the capsule endoscope 300) in introducing the capsule endoscope 300 into the body cavity, and the elastic material can recover and expand to the rod shape which is of the originally fixed shape in releasing the capsule endoscope 300 in the body cavity.

In the above configuration, after the capsule-indwelling-type medical device 600 is introduced into and released at the desired position in the body cavity, the placement position and visual field direction of the capsule endoscope 300 are roughly determined while the latching member 552 is arranged such that the rotating fulcrum 553 is located on the side of the body cavity tissue 411. Then, the latching portion 550 is latched in the body cavity tissue 411 by the endoscope hemostatic clip 410. In this state of things, while the display device 403 is monitored, the placement position and visual field direction of the capsule endoscope 300 are finely adjusted with the endoscopic treatment tool by forcedly applying the external force. In the state where the desired placement position and visual field direction are obtained, the spike-shape latching portion 551 is slightly pulled outward, and the latching portion 551 is directly latched in the body cavity tissue 411 by inserting the latching portion 551 into the body cavity tissue 411 with the endoscopic treatment tool while the tension is applied to the latching portion 535 by the retaining member 501. At this point, the latched state in which the retaining member 501 is not moved with respect to the latching member 552 is achieved unless the external force is forcedly applied. Therefore, the retaining member 501 (capsule endoscope 300) is not rotated with respect to the latching member 236, the retaining member 501 is maintained in the state in which the retaining member 501 comes into line contact with the body cavity tissue 411 while the generatrix L is kept in the desired direction, and the visual field can correctly be obtained. The clipping treatment becomes simplified because the clipping treatment is realized at one point on the side of the latching portion 550.

### (Fourteenth modification)

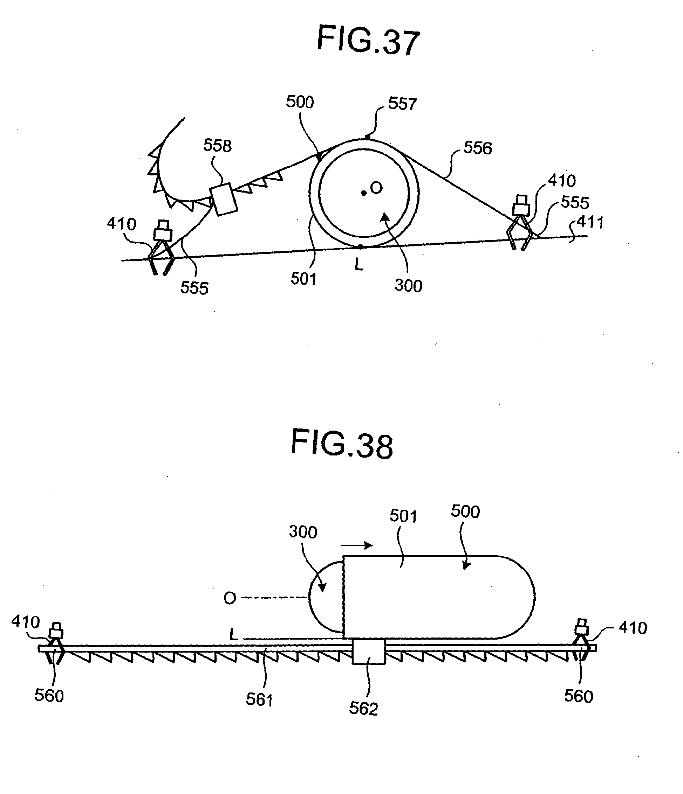

A fourteenth modification will be described with reference to FIG. 37. FIG. 37 is a schematic front view showing a configuration example of the fourteenth modification. In the fourteenth modification, a belt-shape latching member 556 which has latching portions 555 on both ends thereof is provided on the peripheral surface of the retaining member 501 by a joint portion 557 (or rotation fulcrum), and a banding member 558 having the non-return structure is placed in a part of the belt-shape latching member 556. As with the above-described cases, the latching member 556 including the banding member 558 is made of an elastic material, and the latching member 556 can be folded so as to be wound around the retaining member 501.

In the above configuration, after the capsule-indwelling-type medical device 600 is introduced into and released at the desired position in the body cavity, the placement position and visual field direction of the capsule endoscope 300 are roughly determined while the latching member 556 is arranged such that the joint portion 557 is located on the side opposite to the body cavity tissue 411, and the latching portion 555 on one side is latched in the body cavity tissue 411 by the endoscope hemostatic clip 410. Then, the other latching portion 555 is also latched in the body cavity tissue 411 by the endoscope hemostatic clip 410. At this point, in the case where the latching member 556 has slack, the banding member 558 moves in the tight direction, and thereby the tension state of the latching member 556 is adjusted while the tension is applied to the latching member 556 by the retaining member 501. Because the banding member 558 has the non-return structure, the banding member 558 is not freely moved in the lengthwise direction of the belt after the adjustment. Therefore, the strong force for pressing the retaining member 501 (capsule endoscope 300) against the side of the body cavity tissue 411 is also applied by the latching member 556, and the retaining member 501 (capsule endoscope 300) is maintained in the state in which the retaining member 501 comes into line contact with the body cavity tissue 411 while the generatrix L is kept in the desired direction, so that the visual field can correctly be obtained.

### (Sixth embodiment)

A sixth embodiment of the invention will be described with reference to FIG. 38. FIG. 38 is a schematic side view showing a configuration example of the sixth embodiment. The sixth embodiment includes a linear belt-shape guide member which is formed longer than the capsule endoscope 300, and the sixth embodiment includes also includes a latching member 561 having latching portions 560 at both ends thereof. In the latching member 561, the latching portions 560 are latched in the body cavity tissue 411 with tension while aligned with the placement direction of the capsule endoscope 300. A part of the peripheral surface of the retaining member 501 is movably coupled to the latching member 561 by a joint member 562. The latching member 561 has a saw-toothed non-return structure over the whole length thereof, and the joint member 562 is coupled to and latched in the latching member 561 while being movable only in one direction.

According to the configuration of the sixth embodiment, the latching member 561 is positioned on the body cavity tissue 411 while aligned with the placement direction of the capsule endoscope 300, and the latching portions 560 at both the ends of the latching member 561 are latched in the body cavity tissue 411 with the tension, which determines the attitude of the retaining member 501 (i.e., capsule endoscope 300) coupled to the latching member 561 by the joint member 562. That is, the generatrix L of the capsule endoscope 300 which is parallel to the observation axis O comes indirectly into stable line contact with the body cavity tissue 411 in the desired direction through the joint member 562 and the latching member 561, and the indwelling state can be secured such that the orientation of the observation visual field or the observation axis O is not shifted in the observation optical system 307. Because the joint member 562 is movably coupled to the latching member 561, the capsule endoscope 300 is fixed by adjusting the position in the observation axis direction even after the latching member 561 is latched in the body cavity tissue 411.

### (Fifteenth modification)

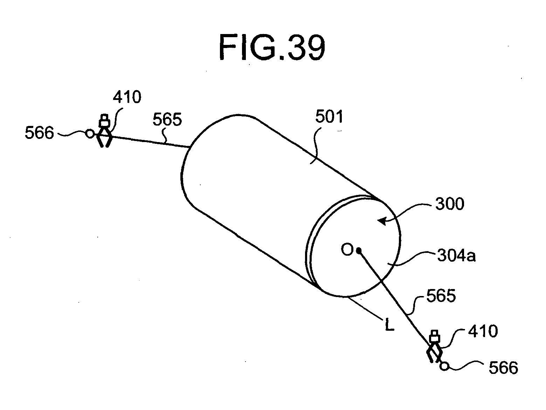

A fifteenth modification will be described with reference to FIG. 39. FIG. 39 is a schematic perspective view showing a configuration example of the fifteenth modification. As with the sixth embodiment, the latching member whose latched positions are set so as to sandwich the capsule endoscope 300 in the axis direction of the observation axis O is used in the fifteenth modification. However, the fifteenth modification differs from the sixth embodiment in that a string-shape latching member 565 is used. That is, the latching member 565 in which a latching portion 566 is provided at an end portion is provided while coupled to a top portion of the front-edge cover chassis 304a of the capsule endoscope 300 or the grasping portion 501a of the retaining member 501, the latching member 565 is positioned while aligned with the placement direction of the capsule endoscope 300. The latching member 565 is also aligned with the placement direction of the capsule endoscope 300, and the latching portion 566 at the end portion is latched in the body cavity tissue 411 by the endoscope hemostatic clip 410 while the latching portion 566 is tensioned.

According to the configuration of the fifteenth modification, the latching member 565 is aligned with the placement direction of the capsule endoscope 300, and the latching portion 566 at the end portion is latched in the body cavity tissue 411 while the latching portion 566 is tensioned. Therefore, the attitudes of the retaining member 501 and capsule endoscope 300 which are coupled to the latching member 565 are also determined. That is, the generatrix L of the capsule endoscope 300 which is parallel to the observation axis O comes into stable line contact with the body cavity tissue 411 in the desired direction, and the indwelling state can be secured such that the orientation of the observation visual field or the observation axis O is not shifted in the observation optical system 307.

The invention is not limited to the above embodiments, but various modifications could be made without departing from the scope of the invention. For example, in the above description, retaining member 501 which mounts and retains the capsule endoscope 300 is included and the latching member is coupled to the retaining member 501. Alternatively, the retaining member 501 may be neglected, and the latching member is directly coupled to the capsule endoscope 300, and thereby the capsule-indwelling-type medical device is configured. Particularly, in the case of the latching members 525 and 530 shown in FIGS. 30 and 32, preferably the latching member is directly coupled to the capsule endoscope 300 without performing the particular process to the capsule endoscope 300.

### INDUSTRIAL APPLICABILITY

The indwelling device for an encapsulated medical device, in vivo indwelling device for a capsule endoscope, and capsule-indwelling-type medical device according to the present invention is useful to the monitoring in the body cavity by using an encapsulated medical device. Particularly the invention is suitable for an application of the general-purpose capsule endoscope for alimentary canal such as capsule endoscope for small intestine to a body-cavity-indwelling capsule endoscope.

## Claims

1. An indwelling device for an encapsulated medical device comprising:
a latching portion for fixing an encapsulated medical device to a body cavity tissue, the encapsulated medical device being introduced into a body cavity of a subject to obtain information of the body cavity, and to wirelessly transmit and output the information of the body cavity to an outside of the subject; and
a retaining unit that is provided with the latching portion to mount and retain the encapsulated medical device.

2. The indwelling device for an encapsulated medical device according to claim 1, wherein the retaining unit has a structure which integrally retains a capsule endoscope as the encapsulated medical device including an observation optical system in a state of being in a surface contact with a peripheral surface of the capsule endoscope.

3. The indwelling device for an encapsulated medical device according to claim 2, wherein the retaining unit includes a substantially cylindrical portion having a radius which is substantially the same as the capsule endoscope.

4. The indwelling device for an encapsulated medical device according to claim 2 or 3, wherein the retaining unit is formed in a cap shape.

5. The indwelling device for an encapsulated medical device according to any one of claims 2 to 4, wherein the retaining unit includes a hood portion for the observation optical system.

6. The indwelling device for an encapsulated medical device according to claim 5, wherein the hood portion is located outside an observation visual field of the observation optical system.

7. The indwelling device for an encapsulated medical device according to claim 5, wherein the hood portion has an abutting portion where the capsule endoscope abuts when mounted.

8. The indwelling device for an encapsulated medical device according to claim 5, wherein the hood portion is formed by a transparent member.

9. The indwelling device for an encapsulated medical device according to claim 8, wherein the hood portion is located within the observation visual field of the observation optical system.

10. The indwelling device for an encapsulated medical device according to claim 8 or 9, wherein the hood portion has a dome shape which covers a whole of the observation visual field of the observation optical system.

11. The indwelling device for an encapsulated medical device according to claim 10, wherein a dirt protective coating is applied to an outer surface of the hood portion.

12. The indwelling device for an encapsulated medical device according to claim 11, wherein the dirt protective coating is a water-repellent coating.

13. The indwelling device for an encapsulated medical device according to claim 11, wherein the dirt protective coating is a hydrophilic coating.

14. The indwelling device for an encapsulated medical device according to any one of claims 10 to 13, wherein the dome shape portion of the hood portion has optical characteristics which functions with respect to the observation optical system.

15. The indwelling device for an encapsulated medical device according to any one of claims 2 to 14, wherein the retaining unit is integral with the latching portion which is formed at a position within the observation visual field of the observation optical system.

16. The indwelling device for an encapsulated medical device according to any one of claims 1 to 14, wherein the latching portion is a clip which is provided so as to be coupled to the retaining unit by a string member, the clip being fixed to a body cavity tissue by an endoscopic treatment tool.

17. The indwelling device for an encapsulated medical device according to any one of claims 1 to 15, wherein the latching portion is a hole portion which is formed so as to be integral with the retaining unit, the hole portion being fixed to the body cavity tissue by an endoscopic fixture.

18. The indwelling device for an encapsulated medical device according to any one of claims 1 to 15, wherein the latching portion is a suction portion which is formed so as to be integral with the retaining unit, the suction portion sucking a part of the body cavity tissue to be fixed by the endoscopic fixture.

19. The indwelling device for an encapsulated medical device according to any one of claims 1 to 14, wherein the latching portion is an annular string member which is provided so as to be coupled to the retaining unit, the annular string member being fixed to the body cavity tissue by the endoscopic fixture.

20. The indwelling device for an encapsulated medical device according to any one of claims 1 to 14, wherein the latching portion is a mesh member which is provided so as to be coupled to the retaining unit, an arbitrary point of the mesh member being fixed to the body cavity tissue by the endoscopic fixture.

21. The indwelling device for an encapsulated medical device according to any one of claims 1 to 20, wherein a plurality of latching portions are provided so as to be located at different points of the retaining member.

22. The indwelling device for an encapsulated medical device according to claim 20, wherein the plurality of latching portions have differences in one of size and shape.

23. A capsule-indwelling-type medical device comprising:
the indwelling device for an encapsulated medical device according to any one of claims 1 to 22; and
an encapsulated medical device that is mounted and retained in a retaining unit of the indwelling device for an encapsulated medical device, introduced into a body cavity, fixed to a body cavity tissue by a latching portion, and obtains information of the body cavity of a subject to wirelessly transmit and output the information of the body cavity to an outside of the subject.

24. An in vivo indwelling device for a capsule endoscope comprising:
a retainer that has an engaging and retaining unit capable of engaging with an exterior of a capsule endoscope to retain the capsule endoscope;
an opening portion that is formed in the retainer so as to be capable of securing an observation visual field of the capsule endoscope retained by the engaging and retaining unit; and
an attachment unit that is provided in an outer surface of the retainer, and can be attached to an organism wall.

25. The in vivo indwelling device for a capsule endoscope according to claim 24, wherein the opening portion is formed at one end portion of the retainer, and
the attachment unit is provided at the other end portion of the retainer.

26. An indwelling device for an encapsulated medical device comprising:
a retaining member that mounts and retains an encapsulated medical device, the encapsulated medical device being introduced into a body cavity to obtain information of a body cavity; and
a latching member that is provided so as to be coupled to the retaining member, and has a plurality of latching portions which are latched to a body cavity tissue with tension at positions located outside a projection plane of the encapsulated medical device with respect to the body cavity tissue, the latching portions holding the encapsulated medical device at the positions.

27. The indwelling device for an encapsulated medical device according to claim 26, wherein the latching member has the plurality of latching portions which are latched to the body cavity tissue so that a generatrix of a capsule endoscope as the encapsulated medical device including an observation optical system comes into line contact with the body cavity tissue in a desired direction.

28. The indwelling device for an encapsulated medical device according to claim 26 or 27, wherein the latching member has the plurality of latching portions whose latching positions are set so as to prevent an orientation of an observation visual field of the observation optical system from deviating.

29. The indwelling device for an encapsulated medical device according to any one of claims 26 to 28, wherein the latching member has the plurality of latching portions whose latching positions are set so as to put an observation axis of the observation optical system therebetween.

30. The indwelling device for an encapsulated medical device according to any one of claims 26 to 29, wherein the latching member has the plurality of latching portions whose latching positions are set so as to offset moments acting on the encapsulated medical device from the respective latching positions.

31. The indwelling device for an encapsulated medical device according to any one of claims 26 to 30, wherein the latching member is formed by an elastic material which allows a flexible folding of the latching member around the retaining member in introducing into the body cavity, and spreading and returning thereof to an original static shape in releasing into the body cavity.

32. The indwelling device for an encapsulated medical device according to any one of claims 26 to 31, wherein the latching member is formed in a grip shape.

33. The indwelling device for an encapsulated medical device according to any one of claims 26 to 31, wherein the latching member is formed in a rod shape.

34. The indwelling device for an encapsulated medical device according to any one of claims 26 to 33, wherein the latching member is coupled so as to be biased to one side face of the retaining member in a tangential manner.

35. The indwelling device for an encapsulated medical device according to any one of claims 26 to 34, wherein the latching member is rotatably coupled to the retaining member.

36. The indwelling device for an encapsulated medical device according to any one of claims 26 to 35, wherein the latching member includes a banding member having a non-return structure.

37. The indwelling device for an encapsulated medical device according to any one of claims 26 to 32, wherein the latching member is formed by a guide member which, having a non-return structure, is arranged in an observation axis of the observation optical system, and to which the retaining member is movably coupled.

38. The indwelling device for an encapsulated medical device according to any one of claims 26 to 37, wherein the latching portion is a portion which is latched to the body cavity tissue by an endoscopic fixture.

39. The indwelling device for an encapsulated medical device according to claim 38, wherein the latching portion is formed in a multistage so that a latching performed by the endoscopic fixture can be freely selected in the latching member.

40. The indwelling device for an encapsulated medical device according to any one of claims 26 to 39, wherein the latching portion includes a latching portion which has a self-latching function with respect to the body cavity tissue, and is directly latched to the body cavity tissue by an endoscopic treatment.

41. A capsule-indwelling-type medical device, comprising:
the indwelling device for an encapsulated medical device according to any one of claims 26 to 40; and
an encapsulated medical device that is mounted to and retained in a retaining member of the indwelling device for an encapsulated medical device, introduced into a body cavity from an oral cavity, fixed to a body cavity tissue by a latching portion of a latching member, and obtains information of the body cavity of a subject to wirelessly transmit and output the information of the body cavity to an outside of the subject.

42. A capsule-indwelling-type medical device, comprising:
an encapsulated medical device that has an observation optical system; and
a latching member that has a plurality of latching portions provided outside a visual field of the observation optical system in the encapsulated medical device, the latching portions attaching the encapsulated medical device to a body cavity tissue.

43. The capsule-indwelling-type medical device according to claim 42, wherein the encapsulated medical device includes a setting portion in a lowermost portion thereof, the setting portion placing the encapsulated medical device to the body cavity tissue, and
the latching member includes a first latching member having a first latching portion which is provided at a position upwardly away from the setting portion in the encapsulated medical device and attached to the body cavity tissue by an attachment member; and a second latching member having a second latching portion which is provided at a position upwardly away from the setting portion and displaced from the first latching portion, the second latching portion being attached to the body cavity tissue by the attachment member.

44. A capsule-indwelling-type medical device, comprising:
an encapsulated medical device that is introduced into a body cavity to obtain information of the body cavity of a subject; and
a latching member that is provided so as to be coupled to the encapsulated medical device, and has a plurality of latching portions which are latched to a body cavity tissue with tension at positions located outside a projection plane of the encapsulated medical device with respect to the body cavity tissue, the latching portions holding the encapsulated medical device at the positions.

45. The capsule-indwelling-type medical device according to claim 44, wherein the encapsulated medical device is a capsule endoscope including an observation optical system.

46. The capsule-indwelling-type medical device according to claim 44 or 45, wherein the latching member has the plurality of latching portions which are latched to the body cavity tissue so that a generatrix of the encapsulated medical device comes into line contact with the body cavity tissue in a desired direction.

47. The capsule-indwelling-type medical device according to any one of claims 44 to 46, wherein the latching member has the plurality of latching portions whose latching positions are set so as to prevent an orientation of an observation visual field of the observation optical system from deviating.

48. The capsule-indwelling-type medical device according to any one of claims 44 to 47, wherein the latching member has the plurality of latching portions whose latching positions are set so as to put an observation axis of the observation optical system therebetween.

49. The capsule-indwelling-type medical device according to any one of claims 44 to 48, wherein the latching member has the plurality of latching portions whose latching portions are set so as to offset moments acting on the encapsulated medical device from the respective latching positions.

50. The capsule-indwelling-type medical device according to any one of claims 44 to 49, wherein the latching member is formed by an elastic material which allows a flexible folding of the latching member around the encapsulated medical device in introducing into the body cavity, and spreading and returning thereof to an original static shape in releasing into the body cavity.

51. The capsule-indwelling-type medical device according to any one of claims 44 to 50, wherein the latching member is formed in a grip shape.

52. The capsule-indwelling-type medical device according to any one of claims 44 to 50, wherein the latching member is formed in a rod shape.

53. The capsule-indwelling-type medical device according to any one of claims 44 to 50, wherein the latching member is formed by a mesh member.

54. The capsule-indwelling-type medical device according to any one of claims 44 to 50, wherein the latching member is formed by a sheet-shape member.

55. The capsule-indwelling-type medical device according to any one of claims 44 to 54, wherein the latching member is coupled so as to be biased to one side face of the encapsulated medical device in a tangential manner.

56. The capsule-indwelling-type medical device according to claim 55, wherein the latching member is arranged so that a joint portion is located on a body cavity tissue side.

57. The capsule-indwelling-type medical device according to claim 55, wherein the latching member is arranged so that a joint portion is located on a side opposite to the body cavity tissue.

58. The capsule-indwelling-type medical device according to any one of claims 44 to 57, wherein the latching member includes a banding member having a non-return structure.

59. The capsule-indwelling-type medical device according to any one of claims 44 to 50, wherein the latching member is formed by a guide member which, having a non-return structure, is arranged in an observation axis of the observation optical system, and to which the encapsulated medical device is movably coupled.

60. The capsule-indwelling-type medical device according to any one of claims 44 to 59, wherein the latching portion is a portion which is latched to the body cavity tissue by an endoscopic fixture.

61. The capsule-indwelling-type medical device according to claim 60, wherein the latching portion is formed in a multistage so that a latching performed by the endoscopic fixture can be freely selected in the latching member.

62. The capsule-indwelling-type medical device according to any one of claims 44 to 61, wherein the latching portion includes a latching portion which has a self-latching function with respect to the body cavity tissue, and is directly latched to the body cavity tissue by an endoscopic treatment.
